(19) Europäisches Patentamt
European Patent Office
Office européen des brevets

(11) EP 3 936 507 A1

(12) **EUROPEAN PATENT APPLICATION**
published in accordance with Art. 153(4) EPC

(43) Date of publication:
**12.01.2022 Bulletin 2022/02**

(21) Application number: **20766035.8**

(22) Date of filing: **06.03.2020**

(51) Int Cl.:
***C07D 495/14*** *(2006.01)* ***A61K 31/55*** *(2006.01)*
***A61P 43/00*** *(2006.01)* ***A61P 35/00*** *(2006.01)*

(86) International application number:
**PCT/CN2020/078187**

(87) International publication number:
**WO 2020/177762 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.03.2019 CN 201910172886**

(71) Applicant: **Medshine Discovery Inc.**
**Nanjing, Jiangsu 210032 (CN)**

(72) Inventors:
- **SHEN, Chunli**
**Shanghai 200131 (CN)**
- **ZHU, Yuchuan**
**Shanghai 200131 (CN)**
- **WANG, Ting**
**Shanghai 200131 (CN)**
- **WU, Chengde**
**Shanghai 200131 (CN)**
- **CHEN, Shuhui**
**Shanghai 200131 (CN)**

(74) Representative: **De Clercq & Partners**
**Edgard Gevaertdreef 10a**
**9830 Sint-Martens-Latem (BE)**

# (54) COMPOUNDS HAVING BOTH EFFECTS OF BET BROMODOMAIN PROTEIN INHIBITION AND PD-L1 GENE REGULATION

(57) Compounds having both BET bromodomain protein inhibitory activity and PD-L1 gene expression regulation, and use thereof in preparing medicaments for treating tumor diseases related to BET bromodomain protein inhibitors and PD-L1 gene expression. Specifically disclosed are a compound as shown in formula (1), and an isomer and a pharmaceutically acceptable salt thereof.

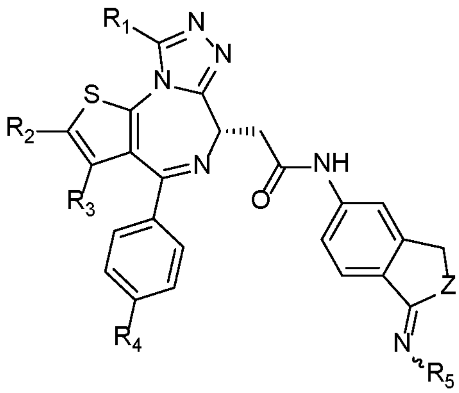

(I)

EP 3 936 507 A1

## Description

### REFERENCE TO RELATED APPLICATIONS

This application claims the priority of CN201910172886.0 filed on March 07, 2019.

### FIELD OF THE INVENTION

The present disclosure relates to a class of compounds that simultaneously inhibit the activity of BET Bromodomain protein and regulate the expression of PD-L1 gene, and use thereof in the manufacture of a medicament for treating diseases related to BET bromodomain protein inhibitors and PD-L1 gene expression. Specifically, the present disclosure relates to a compound as shown in formula (I) and a pharmaceutically acceptable salt thereof.

### BACKGROUND OF THE INVENTION

BET (bromodomain and extraterminal) protein BRD4 is bonded directly with acetylated lysine and other nucleoproteins on the tail of histone via RNA polymerase II (Pol II) to promote gene transcription. BET inhibitors have shown great potential in anti-tumor, and their anti-tumor activity has been confirmed in clinical trials for hematological malignancies. BET inhibitors also have the effect of regulating PD-L1 gene expression and enhancing cytotoxic T cell activity, thereby inhibiting tumor progression in ovarian cancer models.

### SUMMARY OF THE INVENTION

The present disclosure provides a compound as shown in formula (I), or an isomer or a pharmaceutically acceptable salt thereof,

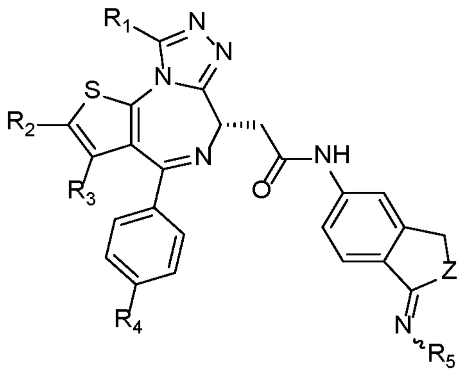

( I )

wherein:

$R_1$, $R_2$ and $R_3$ are each independently selected from $C_{1-3}$ alkyl optionally substituted with 1, 2 or 3 Ra;
$R_4$ is selected from the group consisting of H, F, Cl, Br, I, OH, and $NH_2$;
$R_5$ is selected from the group consisting of H, OH, $NH_2$, CN, $C_{1-6}$ alkyl, $C_{1-6}$ alkylamino, $C_{1-6}$ alkoxy and 4- to 6-membered heterocycloalkyl, wherein said $C_{1-6}$ alkyl, $C_{1-6}$ alkylamino, $C_{1-6}$ alkoxy or 4- to 6-membered heterocycloalkyl is each independently optionally substituted with 1, 2 or 3 $R_b$;
Z is selected from the group consisting of O, $NR_6$ and $CHR_6$;
$R_6$ is selected from the group consisting of H and $C_{1-3}$ alkyl optionally substituted with 1, 2 or 3 $R_c$;
alternatively, $R_5$, $R_6$ and the atoms to which they are attached together form a 5- to 6-membered heterocycloalkenyl or a 5- to 6-membered heteroaryl, wherein the 5- to 6-membered heteroalkenyl or 5- to 6-membered heteroaryl is each independently optionally substituted with 1, 2 or 3 $R_d$;
$R_a$, $R_c$, and $R_d$ are each independently selected from the group consisting of F, Cl, Br, I, OH, $NH_2$, and $CH_3$;
$R_b$ is selected from the group consisting of F, Cl, Br, I, OH, $NH_2$, CN, $C_{1-3}$ alkyl and $C_{1-3}$ alkoxy, wherein the $C_{1-3}$ alkyl or $C_{1-3}$ alkoxy is each independently optionally substituted with 1, 2 or 3 R;
R is each independently selected from the group consisting of F, Cl, Br, I, OH, $NH_2$ and $CH_3$; and

the 4- to 6-membered heterocycloalkyl, 5- to 6-membered heterocycloalkenyl, and 5-to 6-membered heteroaryl each contains 1, 2, 3 or 4 heteroatoms or heteroatom groups independently selected from the group consisting of -NH-, -O-, -S- and N.

In some embodiments disclosed herein, the above-mentioned $R_1$, $R_2$ and $R_3$ are each independently selected from $CH_3$ optionally substituted with 1, 2 or 3 $R_a$, and other variables are as defined herein.

In some embodiments disclosed herein, the above-mentioned $R_1$, $R_2$, and $R_3$ are each independently selected from $CH_3$, and other variables are as defined herein.

In some embodiments disclosed herein, the above-mentioned $R_4$ is selected from Cl, and other variables are as defined herein.

In some embodiments disclosed herein, the above-mentioned $R_b$ is selected from the group consisting of F, Cl, Br, I, OH, $NH_2$, CN, $CH_3$, $CH_2CH_3$, and $OCH_3$, wherein the $CH_3$, $CH_2CH_3$, and $OCH_3$ are optionally substituted with 1, 2, or 3 R, and other variables are as defined herein.

In some embodiments disclosed herein, the above-mentioned $R_b$ is selected from the group consisting of F, Cl, Br, I, OH, $NH_2$, CN, $CH_3$, $CH_2F$, $CHF_2$, $CF_3$, $CH_2CH_3$, and $OCH_3$, and other variables are as defined herein.

In some embodiments disclosed herein, the above-mentioned $R_5$ is selected from the group consisting of H, OH, $NH_2$, CN, $C_{1-3}$ alkyl, $C_{1-3}$ alkylamino, $C_{1-3}$ alkoxy, and oxetanyl, wherein the $C_{1-3}$ alkyl, $C_{1-3}$ alkylamino, $C_{1-3}$ alkoxy, or oxetanyl are each independently optionally substituted with 1, 2, or 3 $R_b$, and other variables are as defined herein.

In some embodiments disclosed herein, the above-mentioned $R_5$ is selected from the group consisting of H, OH, $NH_2$, CN, $CH_3$, $CH_2CH_3$, $CH(CH_3)_2$, $OCH_3$, $OCH_2CH_3$, $NH(CH_3)$ and oxetanyl, wherein the $CH_3$, $CH_2CH_3$, $CH(CH_3)_2$, $OCH_3$, $OCH_2CH_3$, $NH(CH_3)$ or oxetanyl are each independently optionally substituted with 1, 2 or 3 $R_b$, and other variables are as defined herein.

In some embodiments disclosed herein, the above-mentioned $R_5$ is selected from the group consisting of H, OH, $NH_2$, CN, $CH_3$, CH2F, $CHF_2$, $CF_3$, $CH_2CH_3$, $CH(CH_3)_2$, $OCH_3$, $OCH_2F$, $OCHF_2$, $OCF_3$, $OCH_2CH_3$, $OCH(CH_3)_2$, $OCH_2CH_2OCH_3$, $NH(CH_3)$, $N(CH_3)_2$, $NHCH(CH_3)_2$, and

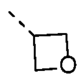,

and other variables are as defined herein.

In some embodiments disclosed herein, the above-mentioned $R_5$, $R_6$ and the atoms to which they are attached together form 4,5-dihydroisoxazolyl, pyrazolyl, pyrrolyl or imidazolyl, wherein the 4,5-dihydroisoxazolyl, pyrazolyl, pyrrolyl or imidazolyl are each independently optionally substituted with 1, 2 or 3 $R_d$.

In some embodiments disclosed herein, the above-mentioned $R_5$, $R_6$ and the atoms to which they are attached together form

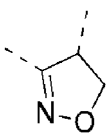

or

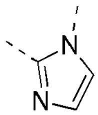,

and other variables are as defined herein.

In some embodiments disclosed herein, the above-mentioned moiety

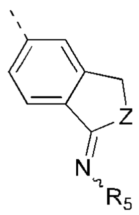
is

selected from the group consisting of

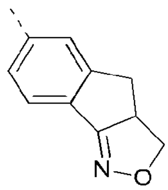

and

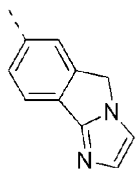
,

and other variables are as defined herein.

The present disclosure also includes some embodiments that are obtained by combining any of the above-mentioned variables.

In some embodiments disclosed herein, the above-mentioned compound, or an isomer or a pharmaceutically acceptable salt thereof is selected from the group consisting of

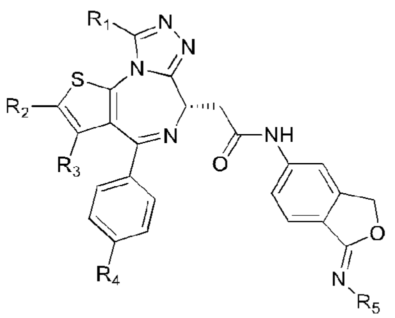
(I-1)

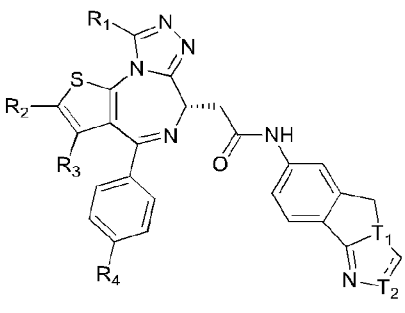
(I-2)

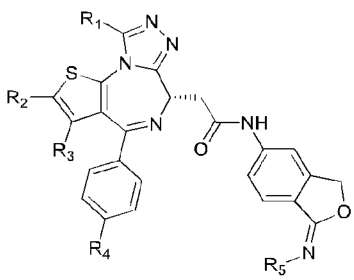
(I-3)

wherein,

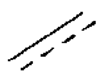

is selected from the group consisting of single bond and double bond;

$R_1$, $R_2$, $R_3$, $R_4$ and $R_5$ are as defined herein;

$T_1$ is CH or N;

$T_2$ is CH, N, or O.

In some embodiments disclosed herein, the above-mentioned compound, or an isomer or a pharmaceutically acceptable salt thereof is selected from the group consisting of

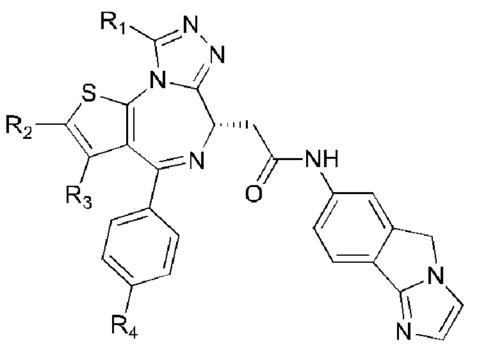

( I -2a)

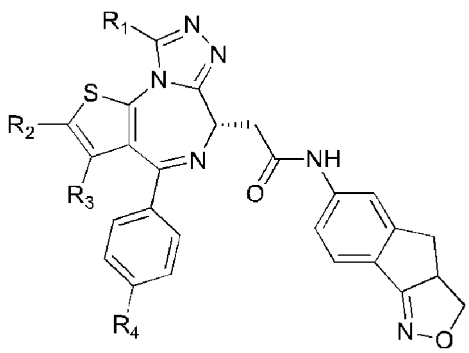

( I -2b)

wherein,

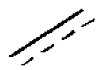

is selected from the group consisting of single bond and double bond;

$R_1$, $R_2$, $R_4$ and $R_3$ are as define herein.

The present disclosure also provides compounds as shown in the formulae below, or isomers or pharmaceutically acceptable salts thereof, wherein the compounds are selected from the group consisting of

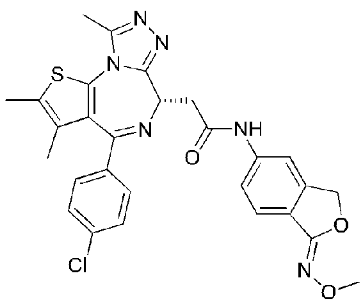

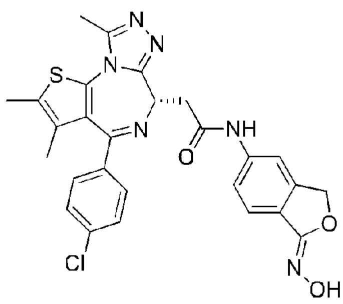

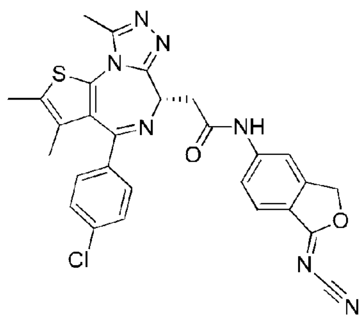

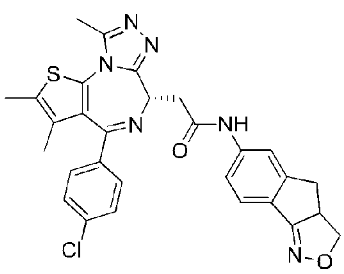

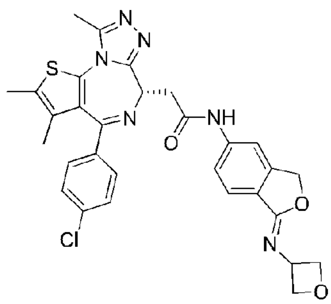

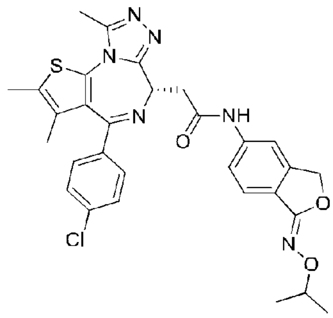

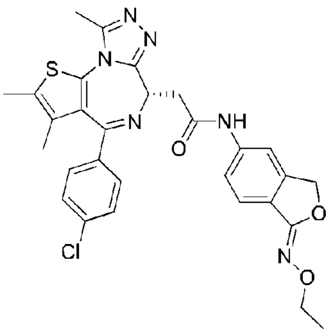
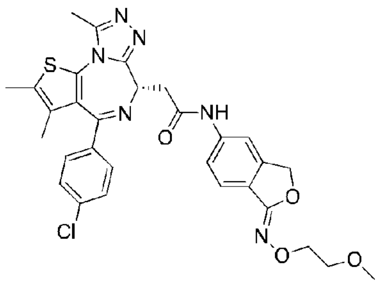
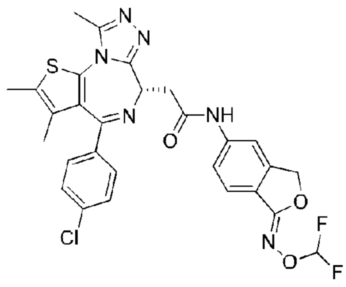

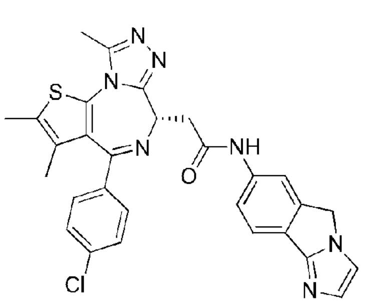
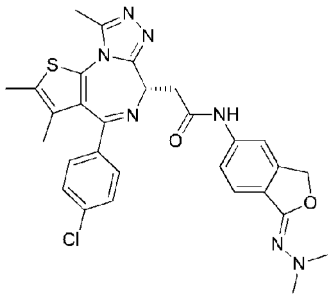
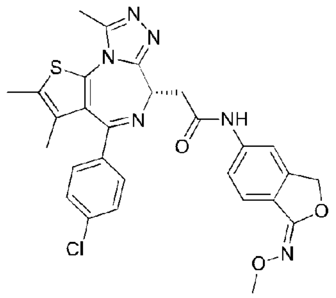

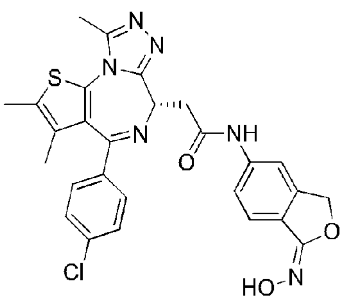
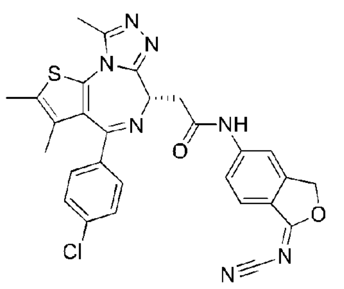
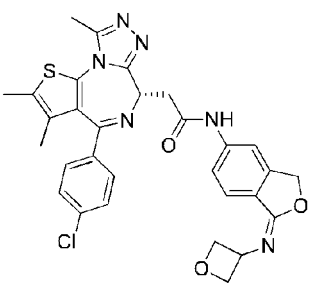

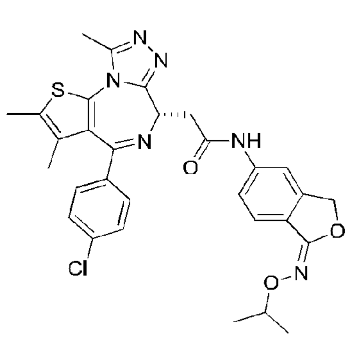
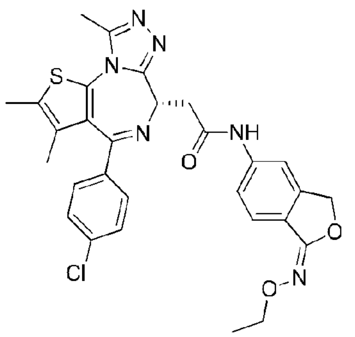
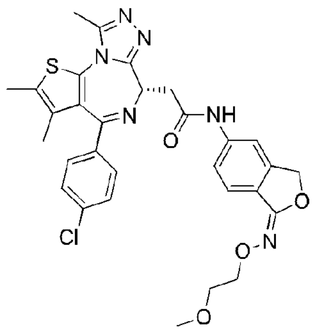

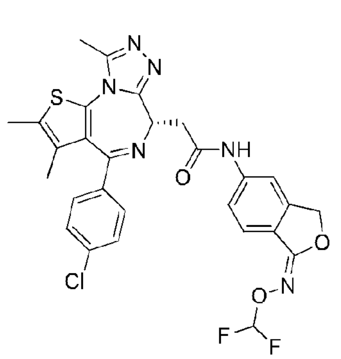
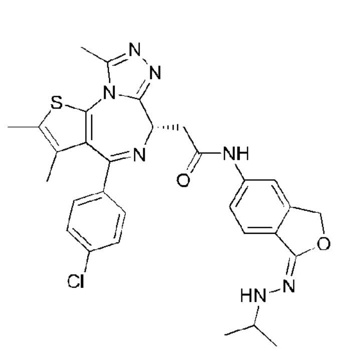
.

In some embodiments disclosed herein, the above-mentioned compound, or an isomer or a pharmaceutically acceptable salt thereof is selected from the group consisting of

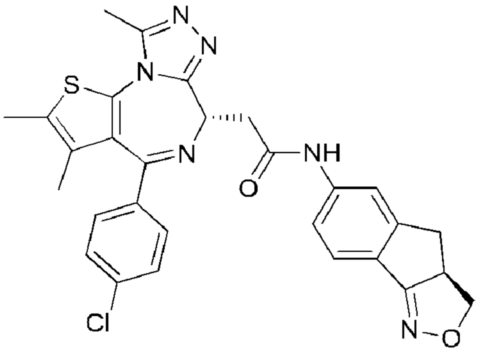

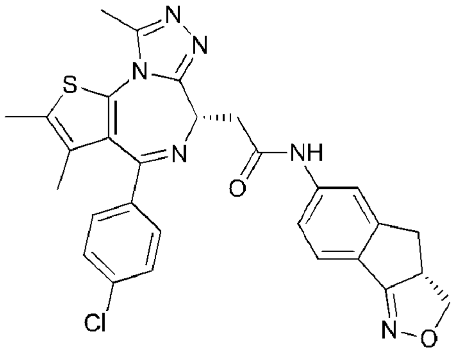
.

The present disclosure also provides a pharmaceutical composition comprising a therapeutically effective amount of the above-mentioned compound, or an isomer or a pharmaceutically acceptable salt thereof, as an active ingredient and a pharmaceutically acceptable carrier.

In some embodiments disclosed herein, the use of the above-mentioned compound, or an isomer or a pharmaceutically acceptable salt thereof, or the above-mentioned composition in the manufacture of a medicament related to BET Bromodomain protein inhibitors and PD-L1 gene expression is provided.

In some embodiments disclosed herein, the above-mentioned medicament is a medicament for treating tumors.

**Definitions and Terms**

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of the compound disclosed herein that is prepared by reacting the compound having a specific substituent disclosed herein with a relatively non-toxic acid or base. When the compound disclosed herein contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound disclosed herein contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

In addition to the salt form, the compound provided herein also exists in prodrug form. The prodrug of the compound described herein is the compound that readily undergoes chemical change under physiological condition to be converted into the compound disclosed herein. Additionally, the prodrug can be converted to the compound disclosed herein by a chemical or biochemical method in vivo environment.

Certain compounds disclosed herein can exist in an unsolvated form or a solvated form, including a hydrated

form. Generally, the solvated form is equivalent to the unsolvated form, and both are encompassed within the scope disclosed herein.

The compound disclosed herein may be present in a specific geometric or stereoisomeric form. The present disclosure contemplates all such compounds, including cis and trans isomer, (-)- and (+)-enantiomer, (R)- and (S)-enantiomer, diastereoisomer, (D)-isomer, (L)-isomer, and racemic mixture and other mixtures, for example, an enantiomer or diastereoisomer enriched mixture, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(D)" or "(+)" means dextroisomer, "(L)" or "(-)" means levoisomer, and "(DL)" or "(±)" means racemate.

Unless otherwise specified, a wedged solid bond (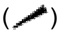) and a wedged dashed bond (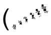) indicate the absolute configuration of a stereocenter; a straight solid bond (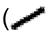) and a straight dashed bond (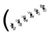) indicate the relative configuration of a stereocenter; a wavy line (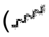) indicates a wedged solid bond (

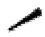

) or a wedged dashed bond (

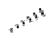

); or a wavy line (

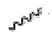

) indicates a straight solid bond (

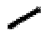

) and a straight dashed bond (

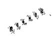

).

Unless otherwise specified, when a double bond structure such as a carbon-carbon double bond, a carbon-nitrogen double bond, and a nitrogen-nitrogen double bond is present in a compound, and each atom on the double bond is attached to two different substituents (in the double bond containing a nitrogen atom, a pair of lone pair electrons on the nitrogen atom is considered as one of the substituents to which it is attached), the compound represents (Z) isomer, (E) isomer, or a mixture of two isomers of the compound, if the atoms on the double bond in the compound are attached to their substituents by a wavy line (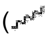

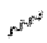

). For example, the compound having following formula (A) means that the compound is present as a single isomer of formula (A-1) or formula (A-2) or as a mixture of two isomers of formula (A-1) and formula (A-2); and the compound

having following formula (B) means that the compound is present as a single isomer of formula (B-1) or formula (B-2) or as a mixture of two isomers of formula (B-1) and formula (B-2). The compound having following formula (C) means that the compound is present as a single isomer of formula (C-1) or formula (C-2) or as a mixture of two isomers of formula (C-1) and formula (C-2).

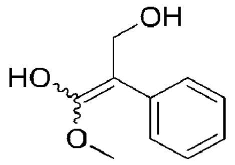 (A) 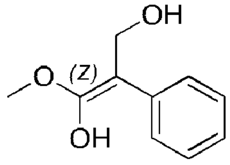 (A-1) 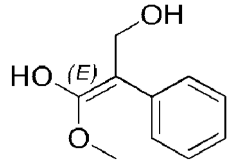 (A-2)

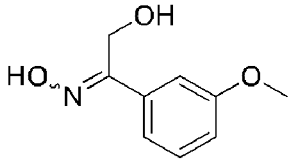 (B) 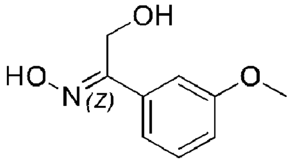 (B-1) 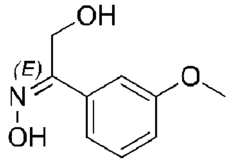 (B-2)

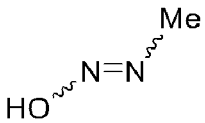 (C) 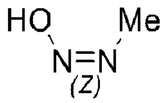 (C-1) 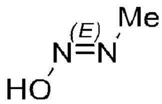 (C-2)

The compounds disclosed herein may be present in a particular form. Unless otherwise specified, the terms "tautomer" or "tautomeric form" means that different functional groups are in dynamic equilibrium at room temperature and can be rapidly converted into each other. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, 98% or more, 99% or more, 99.5% or more, 99.6% or more, 99.7% or more, 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (R)- and (S)-isomer, or D and L isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

The compounds disclosed herein may contain an unnatural proportion of atomic isotopes at one or more of the atoms that make up the compounds. For example, a compound may be labeled with a radioisotope such as tritium ($^3H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite,

that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo.

The term "optionally substituted" means an atom can be substituted by a substituent or not, unless otherwise specified, the species and number of the substituent may be arbitrary as long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variable is a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When an enumerative substituent does not indicate through which atom it is linked to the substituted group, such substituent can be bonded through any of its atoms. For example, a pyridyl group as a substituent may be linked to the substituted group through any one of carbon atoms on the pyridine ring. When an enumerative linking group does not indicate its linking direction, its linking direction is arbitrary. For example, when the linking group L in

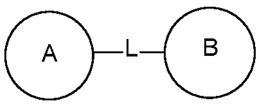

is -M-W-, the -M-W- can be linked to the ring A and the ring B in the same direction as the reading order from left to right to constitute

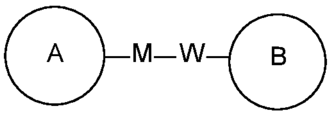

,

or can be linked to the ring A and the ring B in the reverse direction as the reading order from left to right to constitute

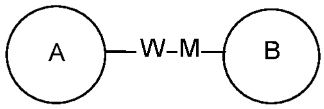

.

A combination of the linking groups, substituents and/or variants thereof is allowed only when such combination can result in a stable compound.

Unless otherwise specified, the term "$C_{1-6}$ alkyl" is used to indicate a linear or branched saturated hydrocarbon group consisting of 1 to 6 carbon atoms. The $C_{1-6}$ alkyl group includes $C_{1-5}$, $C_{1-4}$, $C_{1-3}$, $C_{1-2}$, $C_{2-6}$, $C_{2-4}$, $C_6$, and $C_5$ alkyl groups, and the like. It may be monovalent (e.g., methyl), divalent (e.g., methylene) or multivalent (e.g., methenyl). Examples of $C_{1-6}$ alkyl groups include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, and the like.

Unless otherwise specified, the term "$C_{1-3}$ alkyl" is used to indicate a linear or branched saturated hydrocarbon group consisting of 1 to 3 carbon atoms. The $C_{1-3}$ alkyl group includes $C_{1-2}$ and $C_{2-3}$ alkyl groups and the like. It may be monovalent (e.g., methyl), divalent (e.g., methylene) or multivalent (e.g., methenyl). Examples of $C_{1-3}$ alkyl groups include, but are not limited to, methyl (Me), ethyl (Et), propyl (including n-propyl and isopropyl), and the like.

Unless otherwise specified, the term "$C_{1-6}$ alkoxy" means alkyl groups containing 1 to 6 carbon atoms and attached to the remainder of a molecule by an oxygen atom. The $C_{1-6}$ alkoxy group includes $C_{1-4}$, $C_{1-3}$, $C_{1-2}$, $C_{2-6}$, $C_{2-4}$,

$C_6$, $C_5$, $C_4$, and $C_3$ alkoxy groups, and the like. Examples of $C_{1-6}$ alkoxy groups include, but are not limited to, methoxy, ethoxy, propoxy (including n-propoxy and isopropoxy), butoxy (including n-butoxy, isobutoxy, s-butoxy and t-butoxy), pentoxy (including n-pentoxy, isopropoxy and neopentoxy), hexyloxy, and the like.

Unless otherwise specified, the term "$C_{1-3}$ alkoxy" means alkyl groups containing 1 to 3 carbon atoms and attached to the remainder of a molecule by an oxygen atom. The $C_{1-3}$ alkoxy group includes $C_{1-2}$, $C_{2-3}$, $C_3$, and $C_2$ alkoxy groups, and the like. Examples of $C_{1-3}$ alkoxy groups include, but are not limited to, methoxy, ethoxy, propoxy (including n-propoxy and isopropoxy), and the like.

Unless otherwise specified, the term "$C_{1-6}$ alkylamino" means alkyl groups containing 1 to 6 carbon atoms and attached to the remainder of a molecule by an amino group. The $C_{1-6}$ alkylamino group includes $C_{1-4}$, $C_{1-3}$, $C_{1-2}$, $C_{2-6}$, $C_{2-4}$, $C_6$, $C_5$, $C_4$, $C_3$, and $C_2$ alkylamino groups, and the like. Examples of $C_{1-6}$ alkylamino groups include, but are not limited to $-NHCH_3$, $-N(CH_3)_2$, $-NHCH_2CH_3$, $-N(CH_3)CH_2CH_3$, $-N(CH_2CH_3)(CH_2CH_3)$, $-NHCH_2CH_2CH_3$, $-NHCH_2(CH_3)_2$, $-NHCH_2CH_2CH_2CH_3$, and the like.

Unless otherwise specified, the term "$C_{1-3}$ alkylamino" means alkyl groups containing 1 to 3 carbon atoms and attached to the remainder of a molecule by an amino group. The $C_{1-3}$ alkylamino group includes $C_{1-2}$, $C_3$ and $C_2$ alkylamino groups and the like. Examples of $C_{1-3}$ alkylamino groups include, but are not limited to $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, and the like.

Unless otherwise specified, the term "4- to 6-membered heterocycloalkyl" alone or in combination with other terms each means a saturated cyclic group consisting of 4 to 6 ring atoms, of which 1, 2, 3 or 4 ring atoms are heteroatoms independently selected from O, S and N, and the remainder atoms are carbon atoms, wherein the nitrogen atom is optionally quaternized and the nitrogen and sulfur heteroatoms are optionally oxidized (i.e., NO and $S(O)_p$, p is 1 or 2). The 4- to 6-membered heterocycloalkyl comprises a single ring system and a double ring system, wherein the double ring system comprises a sprio-ring, a fused-ring, and a bridge-ring. In addition, with respect to the "4- to 6-membered heterocycloalkyl", the heteroatom may be present on the position of attachment of the heterocycloalkyl group to the remainder of a molecule. The 4- to 6-membered heterocycloalkyl group includes 5- to 6-membered, 4-membered, 5-membered, and 6-membered heterocycloalkyl groups, and the like. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, or homopiperidinyl and the like.

Unless otherwise specified, the term "5- to 6-membered heterocycloalkenyl" alone or in combination with other terms each means a partially unsaturated cyclic group containing at least one carbon-carbon double bond and consisting of 5 to 6 ring atoms, of which 1, 2, 3 or 4 ring atoms are heteroatoms independently selected from O, S and N, and the remainder atoms are carbon atoms, wherein the nitrogen atom is optionally quaternized and the nitrogen and sulfur heteroatoms are optionally oxidized (i.e., NO and $S(O)_p$, p is 1 or 2). The 4- to 6-membered heterocycloalkenyl comprises a single ring system and a double ring system, wherein the double ring system comprises a sprio-ring, a fused-ring and a bridge-ring, and any ring of the system is non-aromatic. In addition, with respect to the "5- to 6-membered heterocycloalkenyl", the heteroatom may be present on the position of attachment of the heterocycloalkenyl group to the remainder of a molecule. The 5- to 6-membered heterocycloalkenyl group includes 5-membered and 6-membered heterocycloalkenyl groups and the like. Examples of 5- to 6-membered heterocycloalkenyl groups include, but are not limited to

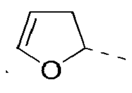 , 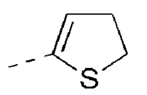 , 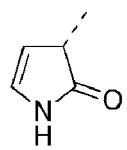 , 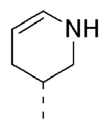 ,

or

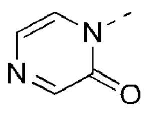 .

Unless otherwise specified, the terms "5- to 6-membered heteroaromatic ring" and "5- to 6-membered heteroaryl" may be used interchangeably. The term "5- to 6-membered heteroaryl" means a monocyclic group having a

conjugated pi electron system and consisting of 5 to 6 ring atoms, of which 1, 2, 3 or 4 ring atoms are heteroatoms independently selected from O, S and N, and the remainder atoms are carbon atoms, wherein the nitrogen atom is optionally quaternized and the nitrogen and sulfur heteroatoms are optionally oxidized (i.e., NO and $S(O)_p$, p is 1 or 2). The 5- to 6-membered heteroaryl group may be attached to the remainder of a molecule by a heteroatom or a carbon atom. The 5- to 6-membered heteroaryl group includes 5-membered and 6-membered heteroaryl groups. Examples of the 5- to 6-membered heteroaryl group include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like).

Unless otherwise specified, $C_{n-n+m}$ or $C_{n-Cn+m}$ includes any one of n to n+m carbons. For example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$. $C_{n-n+m}$ or $C_n$-$C_{n+m}$ also includes any range of n to n+m. For example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$, and the like. Similarly, the n-membered to n+m-membered ring means that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. The n-membered to n+m-membered ring also means that the number of atoms on the ring includes any range from n to n+m. For example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, and the like.

The term "leaving group" refers to a functional group or atom which can be replaced by another functional group or atom through a substitution reaction (such as affinity substitution reaction). For example, representative leaving groups include triflate; chlorine, bromine and iodine; sulfonate group, such as mesylate, tosylate, p-bromobenzenesulfonate, p-toluenesulfonate and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g. acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl such as methyl, ethyl and tert-butyl; acyl such as alkanoyl (e.g. acetyl); arylmethyl such as benzyl (Bn), p-methoxybenzyl (PMB), 9-fluorenylmethyl (Fm), and diphenylmethyl (benzhydryl, DPM); silyl such as trimethylsilyl (TMS) and tert-butyl dimethyl silyl (TBS) and the like.

The compound disclosed herein can be prepared by a variety of synthetic methods well known to the skilled in the art, including the following enumerative embodiment, the embodiment formed by the following enumerative embodiment in combination with other chemical synthesis methods and the equivalent replacement well known to the skilled in the art. The alternative embodiment includes, but is not limited to the embodiment disclosed herein.

The structures of the compounds of the present disclosure can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuK$\alpha$ radiation in a scanning mode of $\varphi/\omega$ scan; after collecting the relevant data, the crystal structure is further analyzed by the direct method (Shelxs97) to confirm the absolute configuration.

All of the solvents used in the present disclosure are commercially available. The present disclosure employs the following abbreviations: aq represents water; HATU represents O-(7-azabenzotriazol-1-yl)-N,N,N',N'-tetramethyluronium hexafluorophosphate; EDC represents N-(3-dimethylaminopropyl)-N'-ethylcarbodiimide hydrochloride; m-CPBA represents 3-chloroperoxybenzoic acid; eq represents equivalent or equivalence; CDI represents carbonyl diimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl, which is an amino protecting group; BOC represents tert-butoxycarbonyl, which is an amino protecting group; HOAc represents acetic acid; $NaCNBH_3$ represents sodium cyanoborohydride; r.t. represents room temperature; O/N represents overnight; THF represents tetrahydrofuran; $Boc_2O$ represents di-tert-butyldicarbonate; TFA represents trifluoroacetic acid; DIPEA represents di-

isopropylethylamine; $SOCl_2$ represents thionyl chloride; $CS_2$ represents carbon disulfide; TsOH represents p-toluenesulfonic acid; NFSI represents N-fluoro-N-(phenylsulfonyl)benzenesulfonamide; NCS represents 1-chloropyrrolidine-2,5-dione; *n*-$Bu_4NF$ represents tetrabutylammonium fluoride; iPrOH represents 2-propanol; mp represents melting point; LDA represents lithium diisopropylamide; LiHMDS represents lithium hexamethyldisilazide; Xantphos represents 4,5-bis(diphenylphosphino)-9,9-dimethylxanthene; $LiAlH_4$ represents lithium aluminum hydride; $Pd_2(dba)_3$ represents tris(dibenzylideneacetone)dipalladium; $Pd(dba)_2$ represents bis(dibenzylideneacetone)palladium; $Pd(dppf)Cl_2$ represents [1,1'-bis(diphenylphosphino)ferrocene]dichloropalladium; $Pd(PPh_3)_4$ represents tetrakis(triphenylphosphine)palladium; IPA represents isopropyl alcohol; DEA represents diethylamine; DMAP represents 4-dimethylaminopyridine; $T_3P$ represents 1-(n-propyl)phosphonic anhydride; TBAI represents tetrabutylammonium iodide; NBS represents N-bromosuccinimide; $POCl_3$ represents phosphorus oxychloride; $NaBH_4$ represents sodium borohydride; $PPh_3$ represents triphenylphosphine; HEPES represents 2-(4-(2-hydroxyethyl)piperazin-1-yl)ethanesulfonic acid; BSA represents bovine serum albumin; CHAPS represents 3-((3-cholamidopropyl)dimethylammonio)-1-propanesulfonic acid; and solutol represents polyethylene glycol-15 hydroxystearate.

Compounds are named according to general naming principles in the art or by ChemDraw® software, and commercially available compounds are named with their vendor directory names.

**Technical effects:**

The present compounds can inhibit the activity of BET Bromodomain and regulate the expression of PD-L1 gene. The present compounds have good PK property, can remarkably down-regulate the expression of PD-L1 gene, and have significant tumor inhibition effect on an animal transplanted tumor model of MC38 mouse colon cancer cells.

**BRIEF DESCRIPTION OF THE DRAWINGS**

Figure 1: Effect of a compound of the present disclosure on PD-L1 expression level in MCF7 cells.
Figure 2: Effect of a compound of the present disclosure on PD-L1 expression level in MDA-MB-231 cells.
Figure 3: Effect of compound 4 on tumor volume of a mouse PAN02 pancreatic cancer tumor model.
Figure 4: Effect of compound 4 on tumor volume of a mouse EMT-6 breast cancer tumor model.

**DETAILED DESCRIPTION OF THE INVENTION**

The present disclosure is described in detail below by means of examples. However, it is not intended that these examples have any disadvantageous limitations to the present disclosure. The present disclosure has been described in detail herein, and the embodiments are also disclosed herein. It will be apparent to those skilled in the art that various changes and modifications may be made to the embodiments disclosed herein without departing from the spirit and scope disclosed herein.

Scheme 1

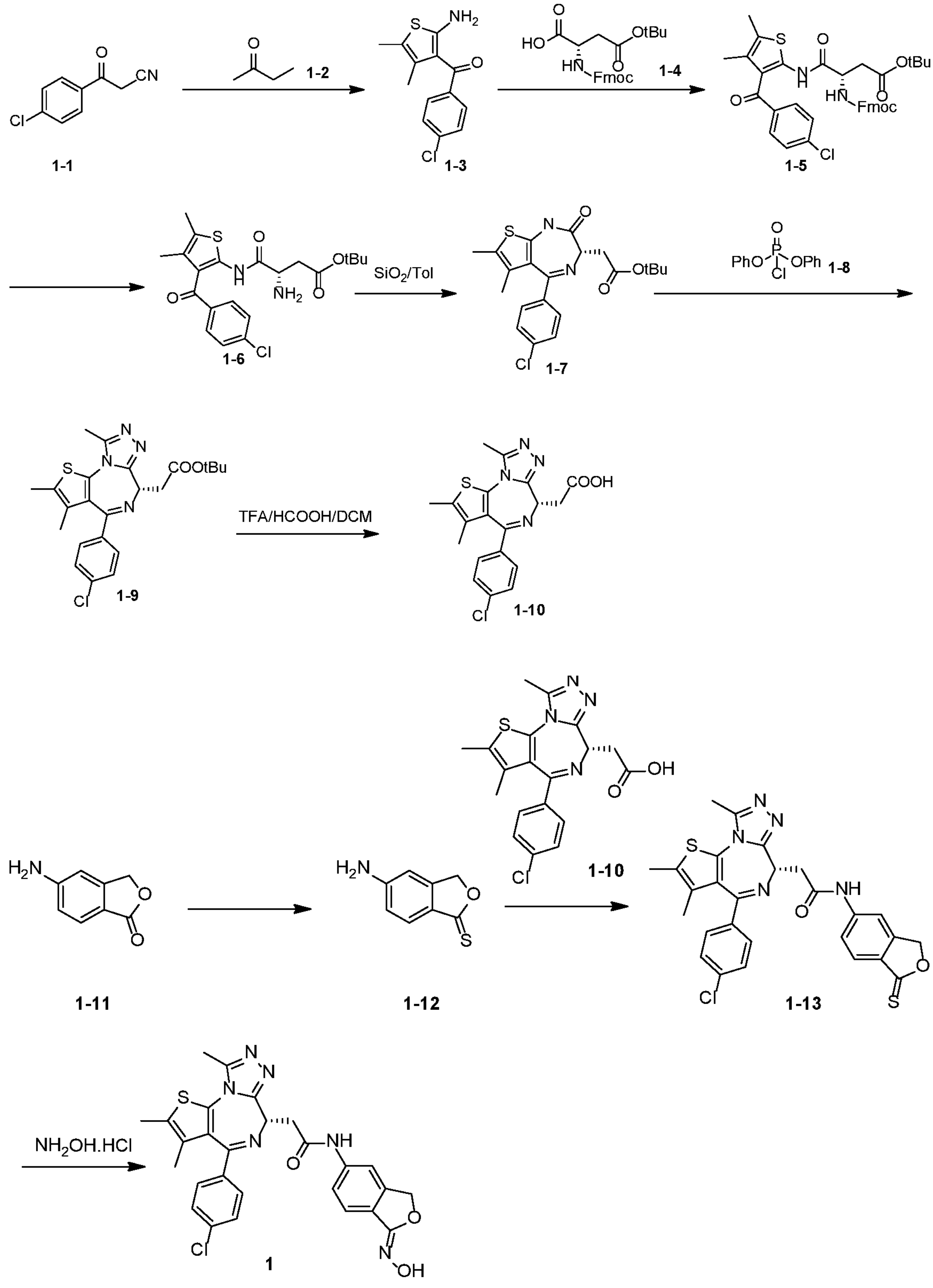


Example 1

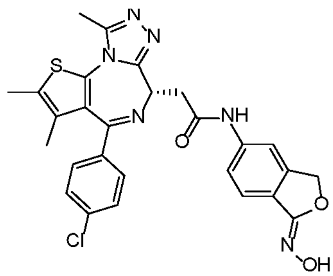

Synthesis of Compound 1-3

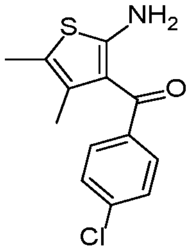

Compound 1-1(850g, 4.73mol, 1eq) was added to a 5L three-necked flask, to which i-PrOH (3400mL) was then added at room temperature. To the reaction system were successively added compound 1-2(344.66g, 4.78mol, 427.62mL, 1.01eq), and morpholine (461.79g, 5.30mol, 466.45mL, 1.12eq). The reaction was heated to 65 °C upon completion of the addition. Sublimed sulfur (160.88g, 5.02mol, 1.06eq) was then added to the reaction system, and the reaction was stirred at 80 °C for 12 hours. TLC and LCMS showed that the starting materials were completely consumed and the target product was produced. The reaction solution was cooled to room temperature and was slowly added to a saturated saline (10L) with stirring to precipitate a solid, which was then filtered. The obtained solid was dissolved in methyl tert-butyl ether (7L), and insoluble substances were filtered off. The filtrate was concentrated under reduced pressure to dryness. A mixture of petroleum ether and ethyl acetate (petroleum ether/ethyl acetate =5/1, 2L) was added to the concentrated dry solid, and the mixture was stirred at room temperature for 10min. The mixture was filtered to afford a solid, which was then dissolved in n-heptane (2L). The resulting mixture was stirred at room temperature for 10min and then filtered to afford Compound 1-3.

$^1$HNMR(400MHz, $CDCl_3$)$\delta$=7.49-7.40(m, 2H), 7.38-7.36(m, 2H), 6.43(brs, 2H), 2.14(s, 3H), 1.56(s, 3H). LCMS(ESI)m/z: 266.0(M+1).

Synthesis of Compound 1-5

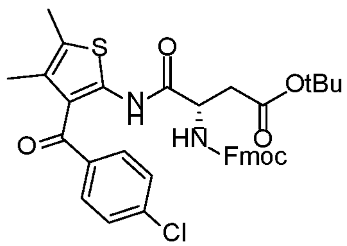

Under nitrogen protection, 1-3(564g, 2.12mol, 1eq) and pyridine (2200mL) were added to a 5L three-necked flask. The reaction was cooled to 0 °C and 1-4(828.94g, 2.02mol, 0.95eq) was added into the reaction. $POCl_3$(390.21g, 2.55mol, 236.49mL, 1.2eq) was added into the reaction at 0-40 °C. After the completion of the dropwise addition, the reaction was further stirred at room temperature (25 °C) for 1 hour. TLC and LCMS showed that the starting materials were completely consumed and the target product was produced. The reaction solution was slowly poured into a solution of 5N HCl (4.5L) with stirring and a solid was precipitated. The resulting solid was filtered and the filter cake was washed with water (2L). The filter cake was dispersed in 5L of water. The mixture was stirred for 20min, and then filtered. The

filter cake was washed with water (2L) to afford Compound 1-5. LCMS(ESI)m/z: 659.0(M+1).

Synthesis of Compound 1-6

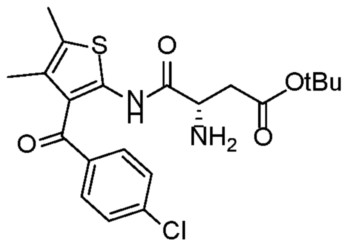

Compound 1-5(2.02kg, 3.07mol, 1eq) and DMF (5.7L) were added to a reaction flask, to which piperidine (621.94g, 7.30mol, 721.34mL, 2.38eq) was added slowly in batches. The reaction was further stirred at room temperature (25 °C) for 2 hours. LCMS showed that the starting materials were completely consumed and the target product was produced. The reaction solution was diluted with toluene (40L). The mixture was washed with water (20L*3) until the pH of the aqueous phase was ~7, and then washed with saturated saline (20L). The organic phase was dried over anhydrous sodium sulfate, and filtered. The filter cake was washed with anhydrous toluene (2L). The filtrates were combined, and 4A molecular sieve was added to the combined filtrate. A 40L toluene solution containing Compound 1-6 was obtained and used directly for the next reaction.

Synthesis of Compound 1-7

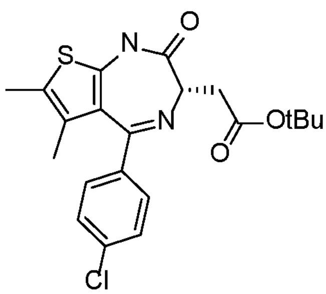

To a 50 L kettle were added the 40L toluene solution containing Compound 1-6 from the above step and then $SiO_2$(2.68kg, 44.62mol, 14.55eq). The reaction was heated to 90 °C and stirred for 12 hours. LCMS showed that the starting materials were completely consumed. The reaction solution was cooled, and filtered. The filtrate was concentrated to afford concentrate 1 (about 900 g). The filter cake obtained in the above step was slurried with 12L of ethyl acetate, filtered, and washed with ethyl acetate. The process was repeated 2 times. The filtrates were combined, and the combined filtrate was concentrated to afford concentrate 2 (about 400 g). The concentrate 1 was slurried with methyl tert-butyl ether (2.7L, 1.8L), and filtered. The filter cake was added to methyl tert-butyl ether (900mL). The mixture was heated to 60 °C, stirred for 1 hour, and then filtered while it was hot. The obtained filter cake was concentrated under reduced pressure to a constant weight to afford the filter cake 1. The concentrate 2 was slurried with methyl tert-butyl ether (800mL), and filtered. The filter cake was added to methyl tert-butyl ether (400mL). The mixture was heated to 60 °C, stirred for 1 hour, and filtered while it was hot (the process was repeated three times). The filter cake was concentrated under reduced pressure to a constant weight to afford the filter cake 2. The resulting filter cake 1 and filter cake 2 was combined to afford Compound 1-7.

$^1$HNMR(400MHz, DMSO-d6)δ=11.20(s, 1H), 7.53-7.50(m, 2H), 7.44-7.42(m, 2H), 3.98-3.94(m, 1H), 3.11-3.07(m, 1H), 2.95-2.89(m, 1H), 2.29(s, 3H), 1.57(s, 3H), 1.40(s, 9H).

Synthesis of Compound 1-9

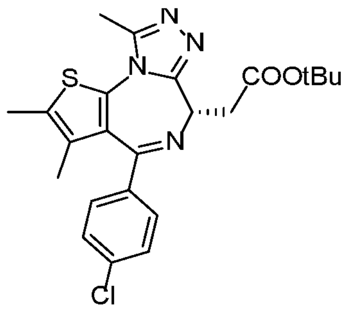

Under nitrogen protection, to a 5L three-necked flask were successively added 1-7 and 2-methyltetrahydrofuran (3360mL). The reaction was cooled to 0 °C. T-BuOK (105.00g, 935.70mmol, 1.4eq) was added to the reaction in batches at 0-5 °C. After the addition, the reaction was further stirred at 0-5 °C for 2 hours. 1-8 (251.36g, 935.70mmol, 193.35mL, 1.4eq) was added to the reaction in batches. After the addition, the reaction was stirred at 0-5 °C for 1 hr. Acetyl hydrazine (99.03g, 1.34mol, 2eq) was added to the reaction and the mixture was stirred at 0-5 °C for 1 hour. Then the reaction was heated to 70 °C and stirred for 12 hours. LCMS showed that the starting materials were not completely consumed and the target product was produced. The reaction solution was diluted with 4L of ethyl acetate, and filtered. The filter cake was washed with 1L of ethyl acetate. Water (4L) was added to the filtrate, and solid sodium bicarbonate was added to adjust the pH of the solution to 8. The layers were separated. The aqueous phase was extracted with ethyl acetate (3L), and the organic phases were combined, dried over anhydrous sodium sulfate, and filtered. The filtrate was concentrated, and the residue was purified by column chromatography to afford Compound 1-9.

$^1$HNMR(400MHz, CDCl3)δ=7.41-7.39(m, 2H), 7.33-7.31(m, 2H), 4.57-4.54(m, 1H), 3.59-3.53(m, 2H), 2.69(s, 3H), 2.14(s, 3H), 1.65(s, 3H), 1.53(s, 9H). LCMS(ESI)m/z: 457.2(M+1).

Synthesis of Compound 1-10

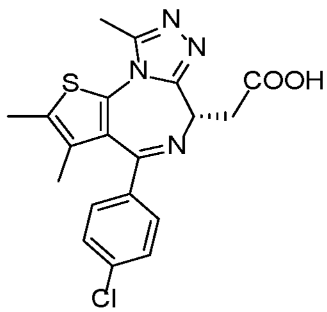

To a reaction flask were added dichloromethane (8mL), compound 1-9(4.6g, 10.07mmol, 1eq), formic acid (8mL), and TFA (13mL). The mixture was reacted at 15 °C for 36 hours. LCMS showed that the starting materials were completely consumed. The reaction solution was concentrated under reduced pressure to dryness. 20mL of water and 30mL of dichloromethane were added to the reaction, and a saturated aqueous solution of sodium bicarbonate was added to adjust the pH of the solution to 5. The layers were separated. The organic phase was washed once with 10mL of saturated aqueous solution of sodium chloride. The organic phase was dried over anhydrous sodium sulfate, and filtered. The filtrate was concentrated to afford a crude product. The crude product was slurried successively with 8mL of ethyl acetate, 8mL of dichloromethane, and 8mL of acetonitrile at room temperature for half an hour to afford Compound 1-10. LCMS(ESI)m/z: 401.1(M+1).

Synthesis of Compound 1-12

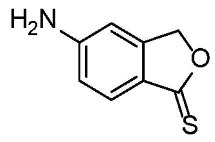

To a flask were successively added toluene (10mL), Compound 1-11(700mg, 4.69mmol, 1eq) and 2,4-bis-(4-methoxyphenyl)-1,3-dithia-2,4-diphosphetan-2,4-disulfide (Lawson's reagent) (949.16mg, 2.35mmol, 0.5eq). The reaction was heated to 110 °C for 18 hours, and then cooled to room temperature. 100mL of water was added to the mixture, and the mixture was extracted with 20mL of ethyl acetate. The combined organic phase was washed once with 10mL of water and once with 10mL of saturated aqueous solution of sodium chloride, respectively. The obtained organic phase was dried over anhydrous sodium sulfate, and filtered. The filtrate was concentrated to afford a crude product. The crude product was purified by column chromatography to afford compound 1-12. LCMS(ESI)m/z: 166.0(M+1).

Synthesis of Compound 1-13

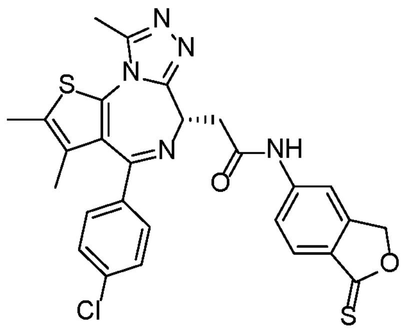

To a reaction flask were added dichloromethane (10mL), Compound 1-12(50mg, 211.85μmol, 1eq), and Compound 1-10(84.93mg, 211.85μmol, 1eq). DMAP(2.59mg, 21.18μmol, 0.1eq), $T_3P$(202.22mg, 317.77μmol, 188.99μL, 50% purity, 1.5eq) and DIPEA(51.94mg, 401.88μmol, 70.00μL, 1.90eq) were added successively to the mixture. The mixture was reacted at 20 °C for 18 hours. To the reaction system was added 10mL of saturated aqueous solution of sodium bicarbonate. The mixture was extracted with 30mL of dichloromethane. The layers were separated. The obtained organic phase was dried over anhydrous sodium sulfate, and filtered. The filtrate was concentrated under reduced pressure to afford a crude product. The crude product was purified by preparative TLC to afford compound 1-13. LCMS(ESI)m/z: 549.1(M+1)

Synthesis of Compound 1

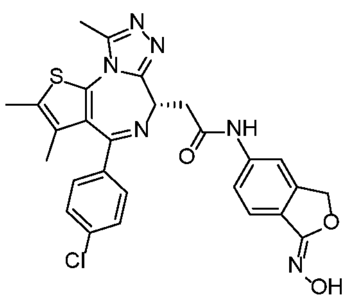

To a reaction flask were added EtOH(10mL), Compound 1-13(100mg, 182.46μmol, 1eq), $NH_2OH.HCl$(38.04mg, 547.37μmol, 3eq), and NaOAc(44.90mg, 547.37μmol, 3eq). The mixture was stirred at 30 °C for 0.5 hours. The reaction solution was concentrated under reduced pressure to dryness and then purified by preparative HPLC (basic) to afford Compound 1.

$^1$HNMR(400MHz, CDCl3)δ=9.59(s, 1H), 7.89(s, 1H), 7.53-7.51(m, 1H), 7.46-7.40(m, 2H), 7.39-7.32(m, 3H), 6.62(brs, 1H), 5.40(s, 2H), 4.64-4.61(m, 1H), 3.87-3.81(m, 1H), 3.52-3.48(m, 1H), 2.70(s, 3H), 2.43(s, 3H), 1.70(s, 3H). LCMS(ESI)m/z: 547.1(M+1).

Scheme 2

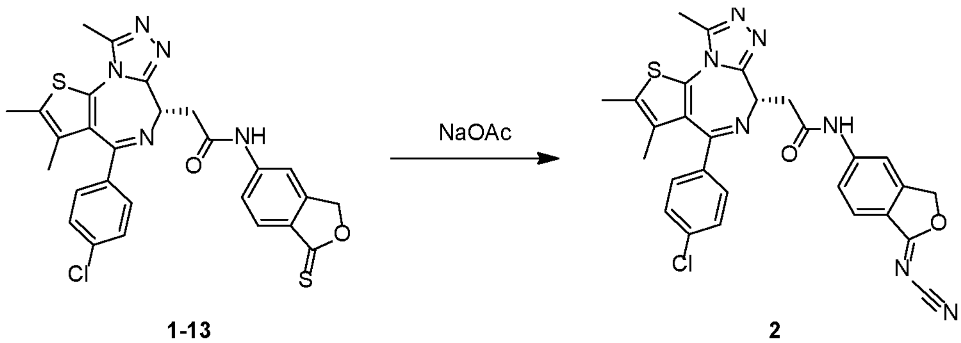


**1-13** **2**

Example 2

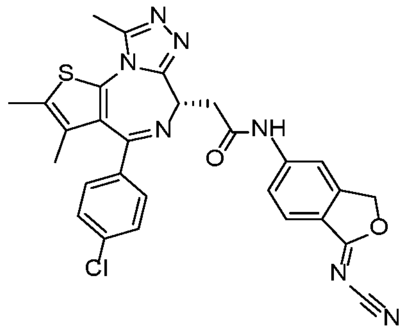

Compound 1-13 (70mg, 127.72μmol, 1eq), EtOH (42mL), sodium acetate (52.39mg, 638.60μmol, 5eq), and $NH_2CN$ (42.95mg, 510.88μmol, 42.95μL, 4eq) were added to a reaction flask, and the mixture was stirred at 75 °C for 20 hours. The reaction solution was concentrated under reduced pressure, and the residue was purified by preparative HPLC (basic) to afford Compound 2.

$^1$HNMR(400MHz, CDCl3)δ=10.18(s, 1H), 8.13(s, 1H), 7.757-7.75(m, 1H), 7.44-7.42(m, 2H), 7.34-7.27(m, 3H), 5.52(s, 2H), 4.62(s, 1H), 3.84-3.82(m, 1H), 3.55-3.53(m, 1H), 2.71(s, 3H), 2.44(s, 3H), 1.72(s, 3H). LCMS(ESI)m/z: 556.1(M+1).

Scheme 3

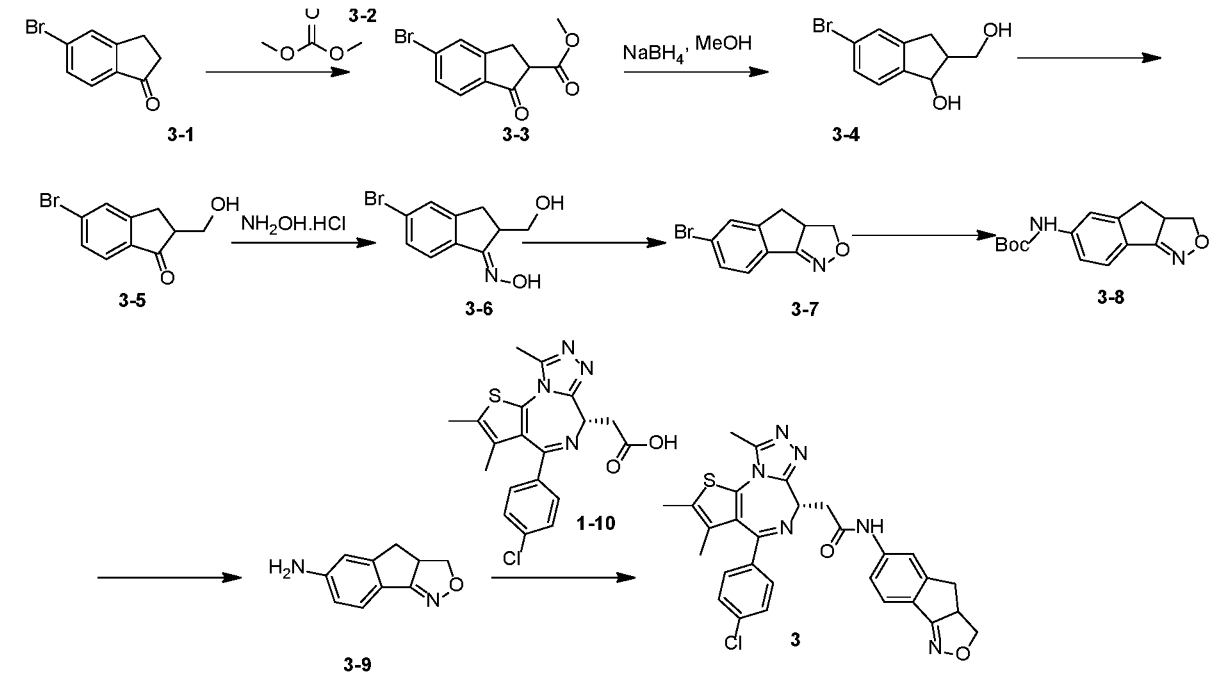


Example 3

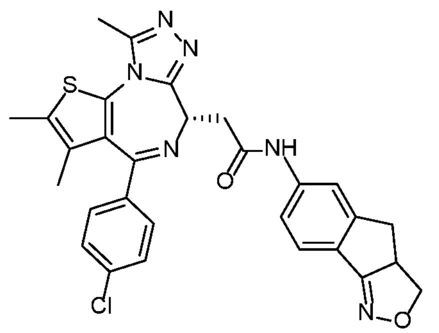

Synthesis of Compound 3-3

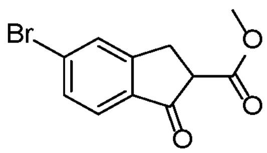

To a reaction flask was added Compound 3-1(6g, 28.43mmol, 1eq) in THF (120mL), followed by NaH(3.41g, 85.29mmol, 60% purity, 3eq) and Compound 3-2(3.84g, 42.64mmol, 3.59mL, 1.5eq). The mixture was reacted at 65 °C for 2 hours. The system was cooled, and 50mL saturated saline was added into the system to quench the reaction. Then 50mL ethyl acetate was added into the system, and the layers were separated. The organic phase was dried over anhydrous sodium sulfate, and filtered. The filtrate was concentrated, and the residue was purified by column chromatography to afford compound 3-3. LCMS(ESI)m/z: 269.0, 270.9 (M+1, M+3).

Synthesis of Compound 3-4

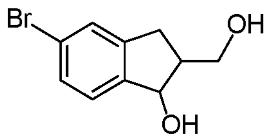

To a reaction flask was added compound 3-3(4g, 14.86mmol, 1eq) in MeOH(40mL), followed by $NaBH_4$(4.50g, 118.92mmol, 8eq). The mixture was reacted at 75 °C for 16 hours. The system was cooled, and then 40mL of saturated ammonium chloride solution was added into the system. The mixture was concentrated. Then 50mL of ethyl acetate was added into the system and the layers were separated. The organic phase was washed with 50mL of saturated saline, dried over anhydrous sodium sulfate, and filtered. The filtrate was concentrated, and the residue was purified by column chromatography to afford compound 3-4. LCMS(ESI)m/z: 241.0, 243.0 (M-1).

Synthesis of Compound 3-5

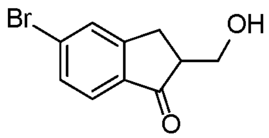

To a reaction flask was added compound 3-4(3g, 12.34mmol, 1eq) in acetone (60mL), and Jones reagent (2.5M, 9.87mL, 2eq) was added to the mixture at -78 °C. The reaction was slowly warmed to 25 °C and stirred for 2 hours. 50mL of isopropanol was added into the system, and the mixture was concentrated. 50mL of water and 50mL of ethyl acetate were added, and the layers were separated. The aqueous phase was extracted with ethyl acetate (50mL*2), and the organic phases were combined. The organic phase was washed with 50mL of saturated saline, dried over anhydrous sodium sulfate, and filtered. The filtrate was concentrated, and the residue was purified by column chromatography to afford Compound 3-5. LCMS(ESI)m/z: 241.0, 243.0 (M+1, M+3).

Synthesis of Compound 3-6

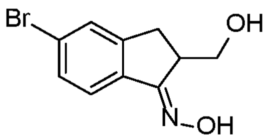

To a reaction flask was added Compound 3-5(1.2g, 4.98mmol, 1eq) in MeOH(24mL), followed by $NH_2OH.HCl$ (518.85mg, 7.47mmol, 1.5eq) and AcONa (612.49mg, 7.47mmol, 1.5eq). The reaction mixture was stirred for 16 hours in an oil bath at 75 °C. The system was concentrated, and then 10mL of water and 10mL of ethyl acetate were added into the system. The layers were separated. The organic phase was washed with saturated saline (10mL*3), dried over anhydrous sodium sulfate, and filtered. The filtrate was concentrated, and the residue was purified by column chromatography to afford Compound 3-6. LCMS(ESI)m/z: 256.0, 258.0 (M+1, M+3).

Synthesis of Compound 3-7

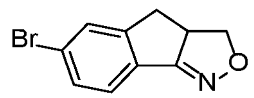

To a reaction flask were added THF(60mL) and Compound 3-6(0.9g, 3.51mmol, 1eq), followed by NaH(702.80mg, 17.57mmol, 60% purity, 5eq) and p-toluenesulfonyl chloride (669.99mg, 3.51mmol, 1eq). The reaction was stirred at 25 °C for 16 hours. Then 10mL of saturated ammonium chloride solution was added into the system to quench the reaction. 10mL of ethyl acetate was then added into the mixture. The layers were separated. The organic phase was washed with 10mL of saturated saline, dried over anhydrous sodium sulfate, and filtered. The filtrate was concentrated, and the residue was purified by column chromatography to afford Compound 3-7. LCMS(ESI)m/z: 237.9, 239.9 (M+1, M+3).

Synthesis of Compound 3-8

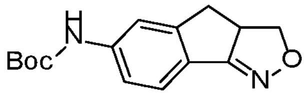

To a reaction flask was added Compound 3-7(0.04g, 168.01μmol, 1eq) in toluene (2mL), followed by tert-butyl carbamate (59.05mg, 504.03μmol, 3eq), $Cs_2CO_3$(136.85mg, 420.03μmol, 2.5eq), Xantphos(19.44mg, 33.60μmol, 0.2eq), and $Pd_2(dba)_3$(15.39mg, 16.80μmol, 0.1eq). The system was purged with nitrogen three times, and the mixture was stirred in an oil bath at 120 °C for 2 hours. To the system was added 10mL of saturated saline. The mixture was extracted with ethyl acetate (10mL*3), and the organic phase was dried over anhydrous sodium sulfate, and filtered. The filtrate was concentrated to afford Compound 3-8. LCMS(ESI)m/z: 275.1 (M+1).

Synthesis of Compound 3-9

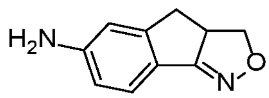

To a reaction flask was added Compound 3-8(0.09g, 328.09μmol, 1eq), and the reaction was cooled to 0 °C. TFA(ImL) that was pre-cooled to 0 °C was then added into the system. After the completion of dropwise addition, the reaction mixture was slowly warmed to 25 °C and stirred for 1 hour. A proper amount of ice pieces was added into the system to quench the reaction. 10mL of ethyl acetate was added into the mixture, and the mixture was adjusted to a pH of 7-8 with saturated sodium bicarbonate solution. The layers were separated after extraction. The organic phase was washed with 10mL of saturated saline, dried over anhydrous sodium sulfate, and filtered. The filtrate was concentrated under reduced pressure to dryness. The residue was purified by preparative TLC to afford Compound 3-9. LCMS(ESI)m/z: 175.1 (M+1).

Synthesis of Compound 3

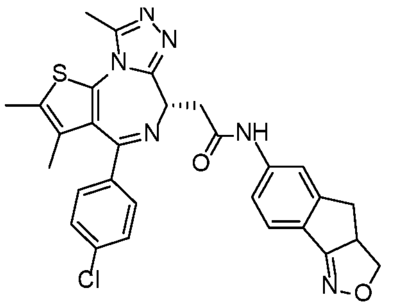

To a reaction flask was added Compound 3-9(14.08mg, 80.82μmol, 1.08eq) in dichloromethane (1mL), followed by Compound 1-10(0.03g, 74.84μmol, 1eq) and DMAP(914.25μg, 7.48μmol, 0.1eq). The system was purged with nitrogen three times, and then DIPEA(13.35mg, 103.27μmol, 17.99μL, 1.38eq), and $T_3P$(33.81mg, 106.27μmol, 31.60μL, 1.42eq, 50% solution in ethyl acetate) were added to the system. The reaction was stirred at 20 °C for 1 hour. To the system were added 5mL of a saturated sodium bicarbonate solution and 5mL of dichloromethane. The layers were separated. The organic phase was washed with 10mL of saturated saline, dried over anhydrous sodium sulfate, and filtered. The filtrate was concentrated, and the residue was purified by preparative HPLC (basic) to afford Compound 3.

$^1$HNMR(400MHz, CDCl3)δ=9.82-9.37(m, 1H), 7.84-7.82(m, 1H), 7.63-7.54(m, 1H), 7.46-7.39(m, 2H), 7.39-7.29(m, 3H), 4.84-4.54(m, 2H), 4.00-3.72(m, 3H), 3.61-3.41(m, 1H), 3.25-3.02(m, 1H), 2.71-2.70(m, 4H), 2.43(s, 3H), 1.71(s, 3H). LCMS(ESI)m/z: 557.2 (M+1).

Scheme 4

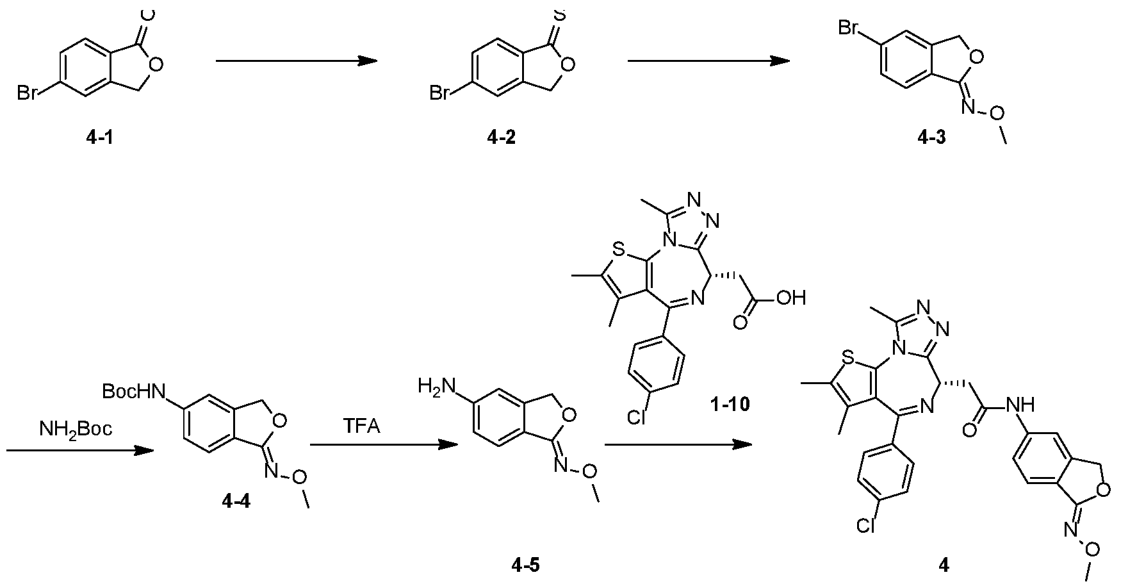


Example 4

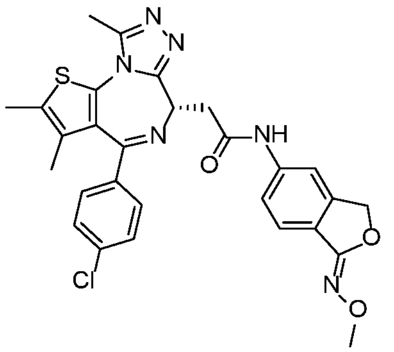

Synthesis of Compound 4-2

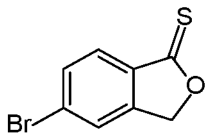

2,4-bis-(4-methoxyphenyl)-1,3-dithia-2,4-diphosphetan-2,4-disulfide (Lawson's reagent) (28.00g, 69.23mmol, 1.47eq) was added to Compound 4-1 (10 g, 46.94mmol, 1eq) in toluene (200mL). The reaction solution was heated to 120 °C and stirred for 12 hours. The reaction solution was cooled to room temperature and a large amount of solid was precipitated. The mixture was filtered, and the filter cake was washed with dichloromethane (20mL*2). The filtrates were combined and concentrated to afford a crude product. The crude product was purified by column chromatography to afford Compound 4-2.

$^1$HNMR(400MHz, CDC13)δ=5.55(s, 2H), 7.65-7.68(m, 2H), 7.87-7.91(m, 1H).

Synthesis of Compound 4-3

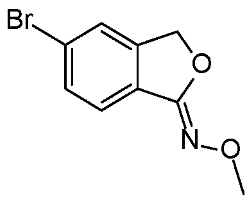

O-methylhydroxylamine hydrochloride (6.5g, 77.83mmol, 5.91mL, 3.24eq) and NaOAc(6.5g, 79.24mmol, 3.30eq) were added to Compound 4-2(5.5g, 24.01mmol, 1eq) in EtOH(100mL). The reaction solution was heated to 90 °C and stirred for 12 hours. The reaction solution was cooled to room temperature and concentrated. The resulting crude product was dissolved in dichloromethane (80mL). The mixture was washed with water (50mL*2). The organic phase was dried over anhydrous sodium sulfate, and filtered. The filtrate was concentrated to afford Compound 4-3. LCMS(ESI)m/z: 241.9, 243.9 (M+1, M+3).

Synthesis of Compound 4-4

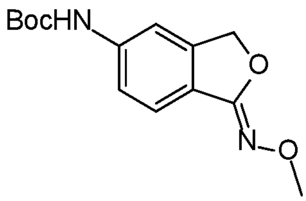

To a solution of Compound 4-3 (5.1g, 21.07mmol, 1eq) and tert-butyl carbamate (3.2g, 27.32mmol, 1.30eq) in toluene (150mL) were successively added $Cs_2CO_3$ (15 g, 46.04 mmol, 2.19eq), $Pd(dba)_2$ (1g, 1.74mmol, 8.25e-2 eq), 4,5-bis diphenyl phosphine 1 g, 1.73mmol, 8.20e-2 eq). The mixture was purged with nitrogen three times, heated to 120 °C, and reacted for 1 hour. The reaction solution was cooled to room temperature, and filtered. The filter cake was washed with ethyl acetate (50mL). The filtrate was concentrated to afford a crude product. The crude product was purified by column chromatography to afford Compound 4-4. LCMS(ESI)m/z: 278.8(M+1).

Synthesis of Compound 4-5

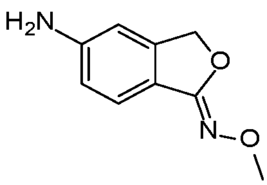

TFA(6.16g, 54.02mmol, 4mL, 4.42eq) was added to a solution of Compound 4-4(3.4g, 12.22mmol, 1eq) in dichloromethane (20mL). The mixture was stirred at room temperature (26 °C) for 0.5 h. Dichloromethane (20mL) and TFA(6.16g, 54.02mmol, 4mL, 4.42eq) were added into the mixture and the reaction was stirred at room temperature (26 °C) for 0.5 h. The reaction solution was adjusted to a pH of 7-8 with saturated aqueous $NaHCO_3$ solution. The reaction solution was allowed to stand, and the layers were separated. The organic phase was washed successively with saturated aqueous $NaHCO_3$ solution (40mL) and water (40mL), dried over anhydrous sodium sulfate, and filtered. The filtrate was concentrated to afford Compound 4-5.

$^1$HNMR(400MHz, CDCl3)$\delta$=3.82(s, 3H), 3.92(s, 2H), 5.25(s, 2H), 6.49(s, 1H), 6.59-6.61(m, 1H), 7.39-7.41(m, 1H). LCMS(ESI)m/z: 178.7(M+1).

Synthesis of Compound 4

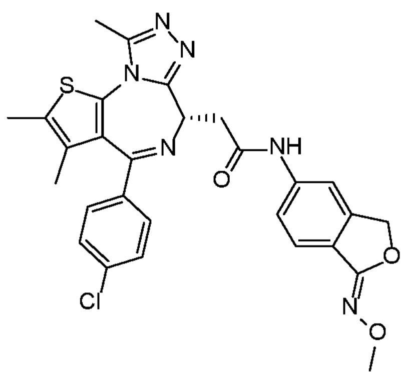

To Compound 1-10(1.8g, 4.49mmol, 1eq) in dichloromethane (50mL) was added DIPEA(742.00mg, 5.74mmol, 1.00mL, 1.28eq). The system was purged with nitrogen three times. $T_3P$(3.75g, 5.89mmol, 3.50mL, 50% purity, 1.31eq), DMAP(500.00mg, 4.09mmol, 9.11e-1 eq), and Compound 4-5(1g, 5.61mmol, 1.25eq) were added into the above reaction solution. The reaction solution was stirred at 30 °C for 1 hour. The reaction solution was then diluted with dichloromethane (20mL) and washed successively with 1 M aqueous HCl solution (50mL*2), saturated aqueous $NaHCO_3$ solution (50mL*2)

and water (50mL*2). The organic phase was dried over anhydrous sodium sulfate, and filtered. The filtrate was concentrated to afford a crude product. The crude product was purified by column chromatography followed by preparative HPLC (basic) to afford Compound 4.

$^1$HNMR(400MHz, CDCl3)$\delta$=1.62-1.96(s, 3H), 2.41(s, 3H), 2.69(s, 3H), 3.503.55(m, 1H), 3.83-3.89(m, 1H), 3.92 (s, 3H), 4.64_4.68(m, 1H), 5.30-5.33(m, 2H), 7.30-7.39(m, 5H), 7.41-7.54(m, 1H), 7.82(s, 1H).9.79(s, 1H). LCMS(ESI)m/z: 561.1(M+1).

Schemes 5&6

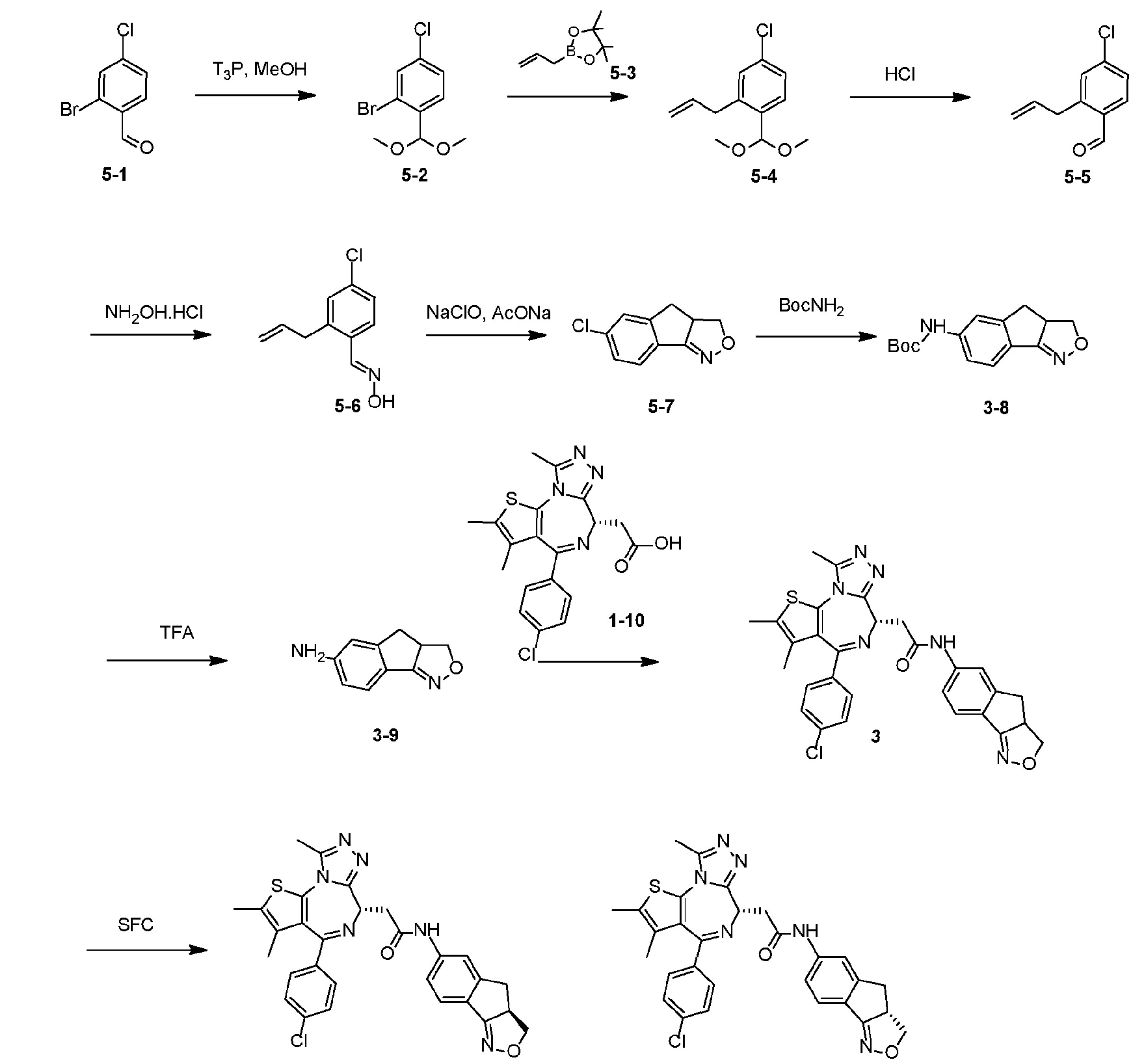


5 or 6

6 or 5

Examples 5&6

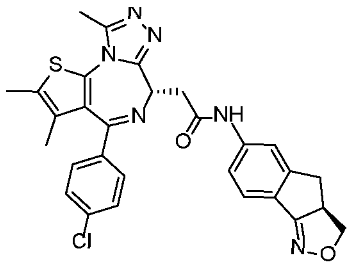

5or6

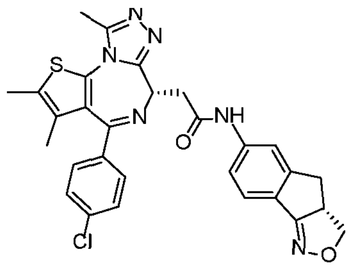

6 or5

Synthesis of Compound 5-2

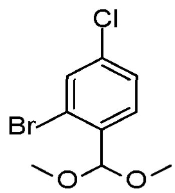

To a reaction flask was added Compound 5-1(30g, 136.70mmol, 1eq) in MeOH(180mL), followed by $T_3P$ (217.47g, 341.74mmol, 203.25mL, 50% purity, 2.5eq). The system was purged 3 times with nitrogen, and the reaction was stirred at room temperature (25 °C) for 16 hours. LCMS and TLC showed that the starting materials disappeared and a new material was produced. Saturated sodium bicarbonate solution was added into the system to adjust the pH to 7-8. The mixture was concentrated under reduced pressure. 200mL of ethyl acetate was added into the system. After extraction, the layers were separated. The organic phase was washed with 200mL of saturated saline, dried over anhydrous sodium sulfate, and filtered. The filtrate was concentrated to afford a crude product. The crude product was purified by column chromatography with neutral alumina of 200-300 meshes to afford Compound 5-2. LCMS(ESI)m/z: 232.9, 234.9(M-31).

Synthesis of Compound 5-4

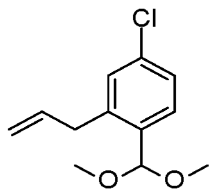

To a reaction flask were added Compound 5-2(33g, 124.28mmol, 1eq) and Compound 5-3(52.21g, 310.70mmol, 2.5eq) in THF(350mL), followed by $H_2O$(90mL), $Na_2CO_3$(26.34g, 248.56mmol, 2eq), and $Pd(dppf)Cl_2$(18.19g, 24.86mmol, 0.2eq). The system was purged three times with nitrogen, and the reaction was stirred in an oil bath at 80 °C for 16 hours. LCMS showed that the starting materials were completely consumed and the target product was produced. The reaction solution was directly used to the next step without further work up. LCMS(ESI)m/z: 227.1(M+1).

Synthesis of Compound 5-5

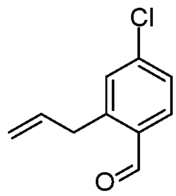

To the reaction solution of Compound 5-4(33g, 145.57mmol, 1eq) from the above step was added dilute HCl solution (1M, 330mL, 2.27eq), and the mixture was stirred at room temperature (25 °C) for 3 hours. LCMS and TLC showed that there were starting materials remaining and a new material was produced. 300mL of ethyl acetate was added into the system. The mixture was emulsified and filtered. The layers were separated, and the organic phase was washed with 300mL of saturated saline, dried over anhydrous sodium sulfate, and filtered. The filtrate was concentrated to afford a crude product. The crude product was purified by column chromatography to afford Compound 5-5.

$^1$HNMR(400MHz, CDCl3)δ=10.23-10.16(m, 1H), 7.79-7.77(m, 1H), 7.57-7.53(m, 1H), 7.38-7.29(m, 1H), 6.03-5.96(m, 1H), 5.15-5.12(m, 1H), 5.06-5.03(dm, 1H), 3.82-3.78(m, 2H). LCMS(ESI)m/z: 181.0(M+1).

Synthesis of Compound 5-6

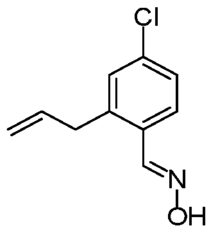

To a reaction flask were added $H_2O$(21mL), $NaHCO_3$ (2.44g, 29.06mmol, 1.13mL, 1.25eq), and $NH_2OH.HCl$(2.02g, 29.06mmol, 1.25eq). Compound 5-5(4.2g, 23.25mmol, 1eq) in MeOH(21mL) was added into the mixture at 0 °C. The reaction was stirred at room temperature (25 °C) for 3 hours. TLC and LCMS showed that the starting materials were completely consumed and the target product was produced. 50mL of water was added into the system. The reaction solution was concentrated. Then 100mL of ethyl acetate was added into the concentrate. The layers were separated. The organic phase was washed with 100mL of saturate saline, dried over anhydrous sodium sulfate, and filtered. The filtrate was concentrated to afford a crude product. The crude product was purified by column chromatography to afford Compound 5-6. LCMS(ESI)m/z: 196.0(M+1).

Synthesis of Compound 5-7

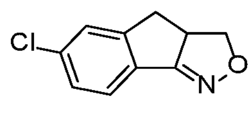

To a reaction flask were successively added 5-6(3.75g, 19.17mmol, 1eq) in dichloromethane (80mL), NaClO(40.77g, 38.33mmol, 33.69mL, 7% purity, 2eq) and AcONa(786.19mg, 9.58mmol, 0.5eq). The mixture was stirred at 25 °C for 16 hours. LCMS and TLC showed that the starting materials were completely consumed and the target product was produced. Then 50mL of dichloromethane and 50mL of water were added into the system. After extraction, the layers were separated. The organic phase was washed with 100mL of saturated ammonium chloride solution, dried over anhydrous sodium sulfate, and filtered. The filtrate was concentrated to afford a crude product. The crude product was purified by column chromatography to afford compound 5-7.

$^1$HNMR(400MHz, CDCl3)δ=7.68-7.66(m, 1H), 7.37-7.33(m, 2H), 4.74-4.70(m, 1H), 4.00-3.94(m, 1H), 3.88-3.82(m, 1H), 3.23-3.17(m, 1H), 2.81-2.75(m, 1H). LCMS(ESI)m/z: 194.0(M+1).

Synthesis of Compound 3-8

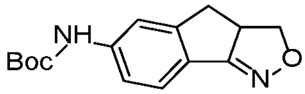

To a reaction flask was added compound 5-7(0.5g, 2.58mmol, 1eq) in 1,4-dioxane (5mL), followed by $Cs_2CO_3$(1.18g, 3.62mmol, 1.4eq), tert-butyl carbamate (363.00mg, 3.10mmol, 1.2eq), 2-dicyclohexylphosphino-2',4',6'-triisopropylbiphenyl (443.16mg, 929.61μmol, 0.36eq), and $Pd(dba)_2$(178.18mg, 309.87μmol, 0.12eq). The system was purged three times with nitrogen. The mixture was stirred in an oil bath at 105 °C for 2.5 hours. LCMS and TLC showed that the starting materials were completely consumed and the target product was produced. The system was cooled, and 20mL of water and 20mL of ethyl acetate were added into the system. The layers were separated. The organic phase was washed with 20mL of saturated saline, dried over anhydrous sodium sulfate, and filtered. The filtrate was concentrated, and the residue was purified by column chromatography to afford Compound 3-8. LCMS(ESI)m/z: 275.1(M+1).

Synthesis of Compound 3-9

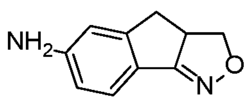

To a reaction flask was added Compound 3-8(0.37g, 1.35mmol, 1eq) in dichloromethane (1mL), and the mixture was cooled to 0 °C. TFA(768.98mg, 6.74mmol, 499.34μL, 5eq) was then added, and the mixture was stirred at room temperature (25 °C) for 5 hours. TLC and LCMS showed that the starting materials were completely consumed and a new material was produced. A proper amount of ice pieces was added into the system to quench the reaction. Then 10mL of ethyl acetate was added to the mixture, and the pH of the mixture was adjusted to be 7-8 by saturated sodium bicarbonate. The layers were separated. The organic phase was washed with 10mL of saturated saline, dried over anhydrous sodium sulfate, and filtered. The filtrate was concentrated, and the residue was purified by column chromatography to afford Compound 3-9.

$^1$HNMR(400MHz, CDCl3)δ=7.52-7.45(m, 1H), 6.81-6.57(m, 2H), 4.60-4.56(m, 1H), 3.91-3.83(m, 1H), 3.73-3.67(m, 1H), 3.07-3.02(m, 1H), 2.73(s, 2H), 2.64(dd, J=7.4, 15.6Hz, 1H). LCMS(ESI)m/z: 175.1(M+1).

Synthesis of Compound 3

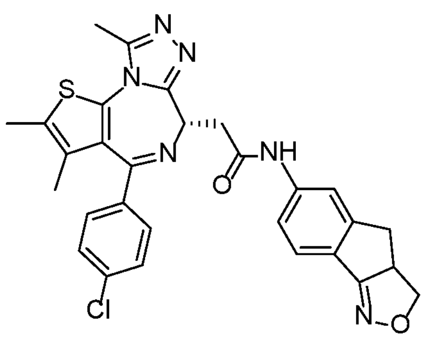

To a reaction flask was added Compound 3-9(0.1g, 574.06μmol, 1.1eq) in dichloromethane (2mL), followed by Compound 1-10(209.21mg, 521.87μmol, 1eq), and DMAP(6.38mg, 52.19μmol, 0.1eq). The system was purged 3 times with nitrogen. To the mixture were successively added DIPEA(93.08mg, 720.18μmol, 125.44μL, 1.38eq), and

$T_3P$(235.79mg, 741.06μmol, 220.36μL, 1.42eq, 50% solution in ethyl acetate). The reaction was stirred at room temperature (25 °C) for 3 hours. LCMS showed that the starting materials were completely consumed and the target product was produced. To the system were added 10mL of a saturated sodium bicarbonate solution, and 10mL of dichloromethane. The layers were separated. The organic phase was washed with 10mL of saturated saline, dried over anhydrous sodium sulfate, and filtered. The filtrate was concentrated to afford a crude product. The crude product was purified by preparative HPLC (basic) to afford Compound 3. LCMS(ESI)m/z: 557.2(M+1).

Synthesis of Compounds 5&6

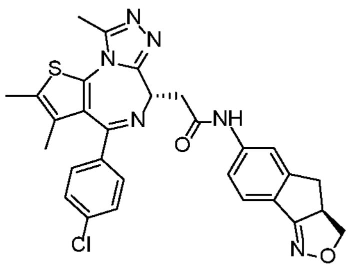

5 or 6

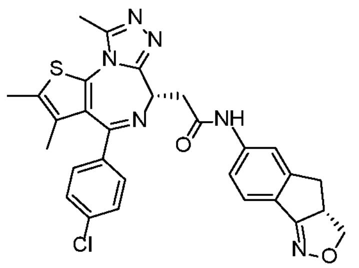

6 or 5

Compound 3(0.18g, 323.12μmol, 1eq) was subjected to SFC (chromatography column: DAICEL CHIRALPAK AS (250mm*50mm, 10 μm); mobile phase: [neutral-MeOH]; B (methanol)%: 40%-40%, 12 min) to be separated into two single configuration compounds. Compound 5(Rt=1.893min) and compound 6(Rt=2.22min) were afforded.

Compound 5(Rt=1.893min)

$^1$HNMR(400MHz, CDCl3)δ=9.60(s, 1H), 7.82(s, 1H), 7.57-7.55(m, 1H), 7.43-7.41(m, 2H), 7.34-7.32(m, 2H), 7.28(brs, 1H), 4.70-4.65(m, 2H), 3.9-3.82(m, 3H), 3.52-3.47(m, 1H), 3.10-3.09(m, 1H), 2.71-2.66(m, 4H), 2.43(s, 3H), 1.71(s, 3H).

LCMS(ESI)m/z: 557.2(M+1).

Compound 6(Rt=2.22min)

$^1$HNMR(400MHz, CDC13)δ=9.55(s, 1H), 7.79(s, 1H), 7.62-7.60(m, 1H), 7.43-7.40(m, 2H), 7.38-7.32(m, 2H), 7.29(brs, 1H), 4.68-4.62(m, 2H), 3.90-3.79(m, 3H), 3.53-3.50(m, 1H), 3.13-3.11(m, 1H), 2.73-2.70(m, 4H), 2.42(s, 3H), 1.70(s, 3H).

LCMS(ESI)m/z: 557.2(M+1).

Scheme 7

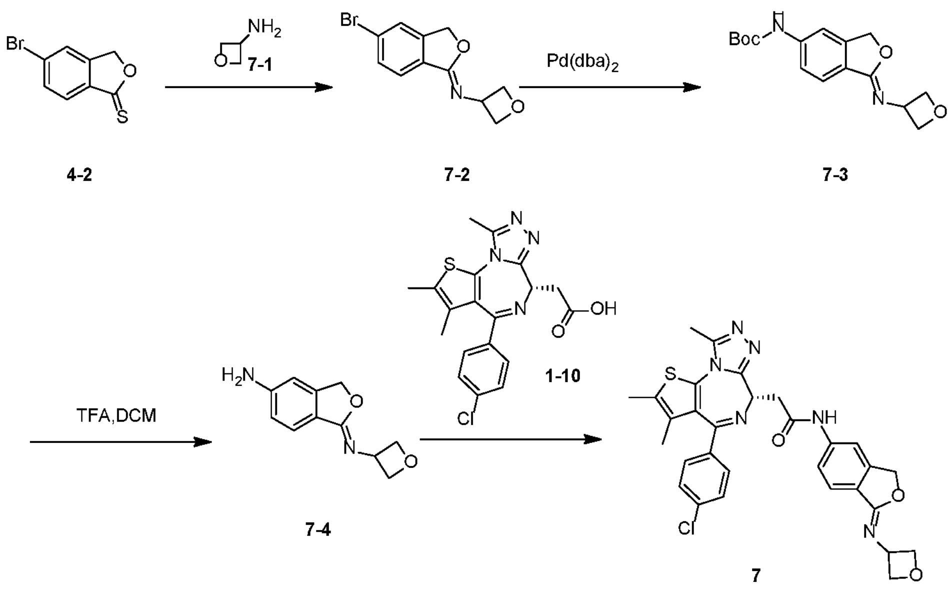


Example 7

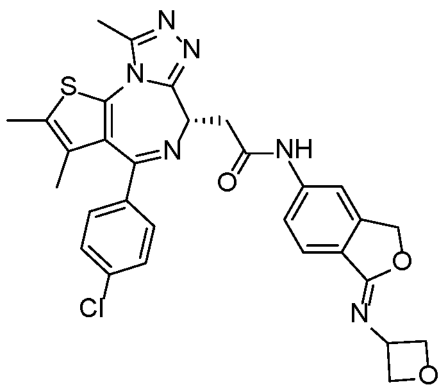

Synthesis of Compound 7-2

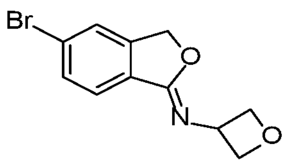

To a reaction flask were added Compound 4-2(0.3g, 1.31mmol, 1eq) in EtOH(3mL) and Compound 7-1(215.19mg, 1.96mmol, 1.5eq), followed by AcONa(322.27mg, 3.93mmol, 3eq). The reaction was stirred at room temperature (25 °C) for 3 hours. TLC showed that the starting points disappeared and a new point appeared. The system was concentrated to afford a crude product. The crude product was purified by column chromatography to afford Compound 7-2. LCMS(ESI)m/z: 267.9, 270.0 (M+1, M+3).

Synthesis of Compound 7-3

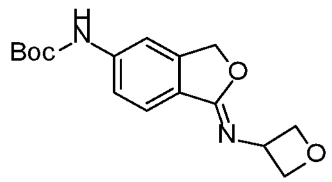

To a reaction flask was added Compound 7-2(0.18g, 671.38μmol, 1eq) in 1,4-dioxane (2mL). To the reaction flask were successively added tert-butyl carbamate (235.95mg, 2.01mmol, 3eq), $Cs_2CO_3$(546.87mg, 1.68mmol, 2.5eq), 2-dicyclohexylphosphino-2',4',6'-triisopropylbiphenyl (64.01mg, 134.28μmol, 0.2eq), and $Pd(dba)_2$(38.60mg, 67.14μmol, 0.1eq). The system was purged three times with nitrogen. The reaction was heated to 105 °C and stirred for 3 hours. LCMS and TLC showed that the starting materials were completely consumed and the target product was produced. To the system were added 10mL of water and 10mL of ethyl acetate. After extraction, the layers were separated. The organic phase was washed with 10mL of saturated saline, dried over anhydrous sodium sulfate, and filtered. The filtrate was concentrated, and the residue was purified by column chromatography to afford Compound 7-3. LCMS(ESI)m/z: 305.1(M+1).

Synthesis of Compound 7-4

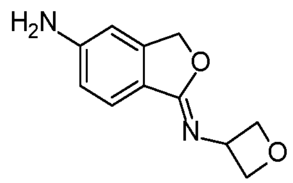

To a reaction flask was added Compound 7-3(0.1g, 328.58μmol, 1eq) in dichloromethane (1mL), and the mixture was cooled to 0 °C. TFA (1.54g, 13.51mmol, 1mL, 41.10eq) that was pre-cooled to 0 °C was added. The reaction was warmed to room temperature (25 °C) and stirred for 2 hours. TLC showed that the starting points disappeared and a new point appeared. A proper amount of ice pieces was added into the system to quench the reaction, and then saturated sodium bicarbonate solution was added to adjust the pH to be 7-8. 10mL of dichloromethane was added to the mixture. After extraction, the layers were separated. The obtained organic phase was washed with 10mL of saturated saline, dried over anhydrous sodium sulfate, and filtered. The filtrate was concentrated under reduced pressure. The residue was purified by column chromatography to afford Compound 7-4. LCMS(ESI)m/z: 205.1(M+1).

Synthesis of Compound 7

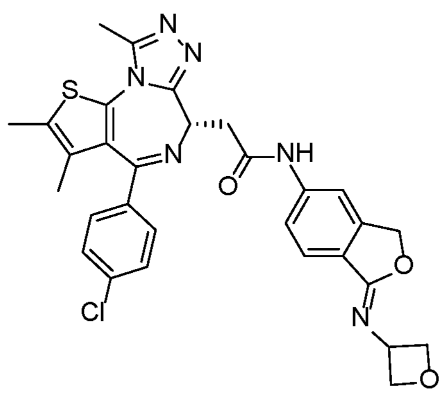

To a reaction flask was added Compound 7-4(25.00mg, 122.41μmol, 1.1eq) in dichloromethane (1mL), followed by Compound 1-10(44.61mg, 111.29μmol, 1eq), and DMAP(1.36mg, 11.13μmol, 0.1eq). The system was purged three times with nitrogen. To the reaction were successively added DIPEA(19.85mg, 153.57μmol, 26.75μL, 1.38eq), and

$T_3P$(100.56mg, 158.03μmol, 93.98μL, 50% purity, 1.42eq). The reaction was stirred at room temperature (25 °C) for 3 hours. LCMS showed that the starting materials were completely consumed and the target product was produced. To the system were added 10mL of saturated sodium bicarbonate solution and 10mL of dichloromethane. After extraction, the layers were separated. The obtained organic phase was washed with 10mL of saturated salt, dried over anhydrous sodium sulfate, and filtered. The filtrate was concentrated under reduced pressure to afford a crude product. The crude product was purified by preparative HPLC (basic) to afford Compound 7.

$^1$HNMR(400MHz, CDCl3)δ=9.60(s, 1H), 7.88(s, 1H), 7.73-7.71(m, 1H), 7.44-7.42(m, 2H), 7.37-7.33(m, 3H), 5.20(s, 2H), 5.01-4.93(m, 3H), 4.76-4.73(m, 2H), 4.64-4.62(m, 1H), 3.87-3.81(m, 1H), 3.53-3.48(m, 1H), 2.70(s, 3H), 2.43(s, 3H), 1.71(s, 3H). LCMS(ESI)m/z: 587.2(M+1).

Scheme 8

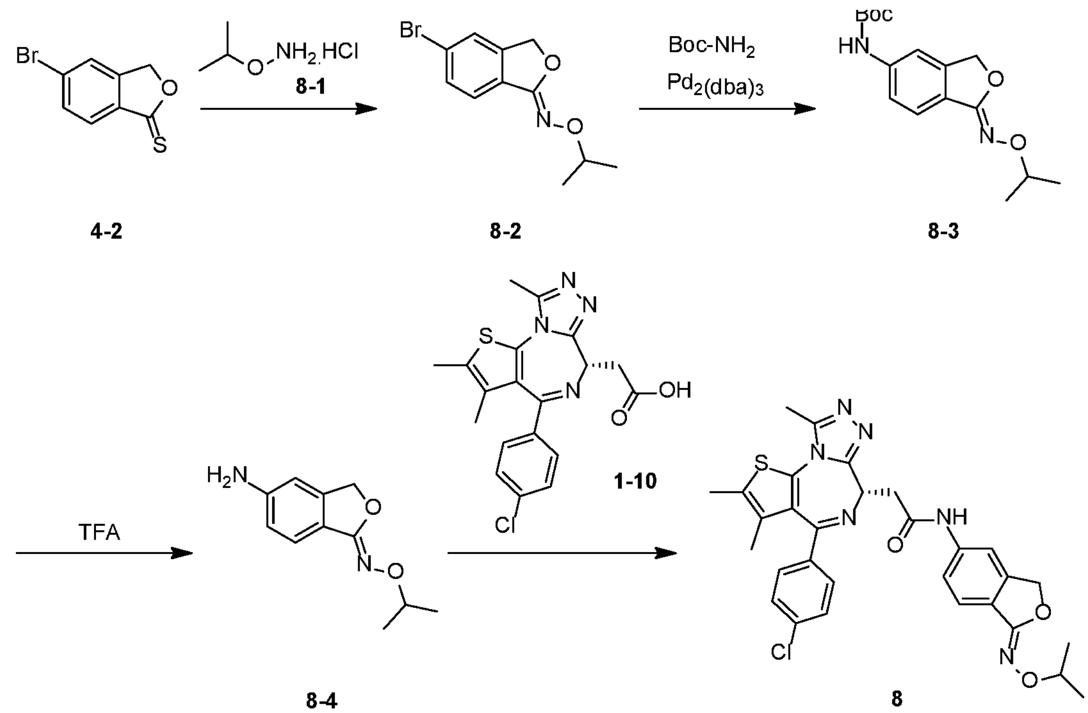


4-2 8-2 8-3 8-4 8

Example 8

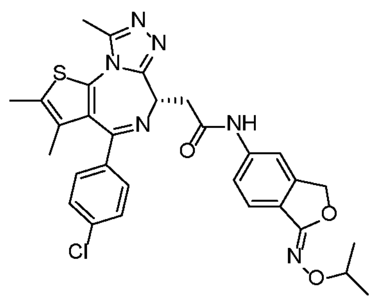

Synthesis of Compound 8-2

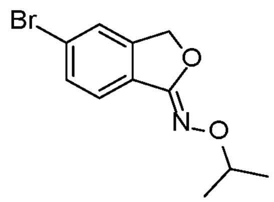

To a reaction flask were added Compound 4-2(650mg, 2.84mmol, 1eq), EtOH(6.5mL), Compound 8-1(411.52mg, 3.69mmol, 1.3eq), and AcONa(698.22mg, 8.51mmol, 3eq). The reaction was stirred at 25 °C for 15 hours. TLC showed that the starting points disappeared and a new point appeared. The system was filtered, and the filtrate was collected and concentrated to dryness under reduced pressure. Water (30mL) was added to the residue. The mixture was extracted with dichloromethane (10mL*5). The obtained mixture was extracted. The organic phase was collected and concentrated under reduced pressure. The residue was mixed with silica gel and purified by column chromatography to afford Compound 8-2. LCMS(ESI)m/z: 270.0, 272.0(M+1, M+3).

Synthesis of Compound 8-3

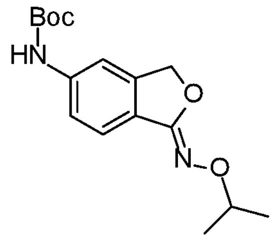

To a reaction flask were added Compound 8-2(535mg, 1.98mmol, 1eq), tert-butyl carbamate (696.05mg, 5.94mmol, 3eq), $Cs_2CO_3$(1.61g, 4.95mmol, 2.5eq), Xantphos(114.60mg, 198.06μmol, 0.1eq), and toluene (5.5mL). The system was purged three times with nitrogen, and then $Pd_2(dba)_3$(181.37mg, 198.06μmol, 0.1eq) was added. The system was purged three times with nitrogen again. The reaction was stirred at 120 °C for 15 hours. LCMS showed that the starting materials were completely consumed and the target product was produced. The reaction solution was filtered, and the filtrate was collected. The filtrate was concentrated under reduced pressure. The residue was mixed with silica gel and purified by column chromatography to afford Compound 8-3. LCMS(ESI)m/z: 307.1(M+1).

Synthesis of Compound 8-4

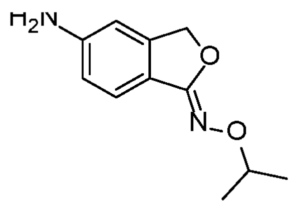

To a reaction flask were added Compound 8-3(470.00mg, 1.53mmol, 1eq), TFA(10mL), and dichloromethane (10mL). The reaction was stirred at 25 °C for 2 hours. TLC showed that the starting points disappeared and a new point appeared. To the system was added a saturated aqueous sodium bicarbonate solution until pH reached 8. The mixture was extracted with dichloromethane (10mL*5). The organic phase was collected and concentrated to dryness under reduced pressure to afford Compound 8-4, which was directly used for the next step.

Synthesis of Compound 8

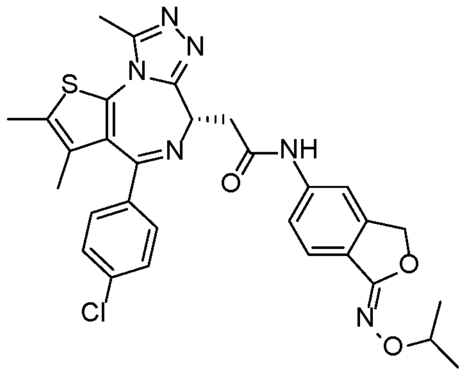

To a reaction flask were added Compound 8-4(66.88mg, 324.29μmol, 1.3eq), Compound 1-10(100mg, 249.45μmol, 1eq), dichloromethane (6mL), DMAP(3.05mg, 24.95μmol, 0.1eq), DIPEA(41.91mg, 324.29μmol, 56.48μL, 1.3eq), and $T_3P$(111.12mg, 349.23μmol, 103.85μL, 1.4eq, 50% solution in ethyl acetate). The reaction was stirred at 25 °C for 2 hours. LCMS showed the disappearance of starting material peaks and the appearance of target product peaks. Water (40mL) was added to the system. The mixture was extracted with dichloromethane (10mL*5). The organic phase was collected, concentrated under reduced pressure to dryness. The residue was purified by preparative HPLC (basic) to afford Compound 8.

$^1$HNMR(400MHz, CDCl3)δ=9.36(s, 1H), 7.86(s, 1H), 7.60-7.58(m, 1H), 7.44-7.42(m, 2H), 7.37-7.33(m, 3H), 5.38(s, 2H), 4.62-4.60(m, 1H), 4.34-4.31(m, 1H), 3.81-3.78(m, 1H), 3.52-3.48(m, 1H), 2.69(s, 3H), 2.42(s, 3H), 1.70(s, 3H), 1.34(d, J=6.3Hz, 6H). LCMS(ESI)m/z: 589.3(M+1).

Scheme 9

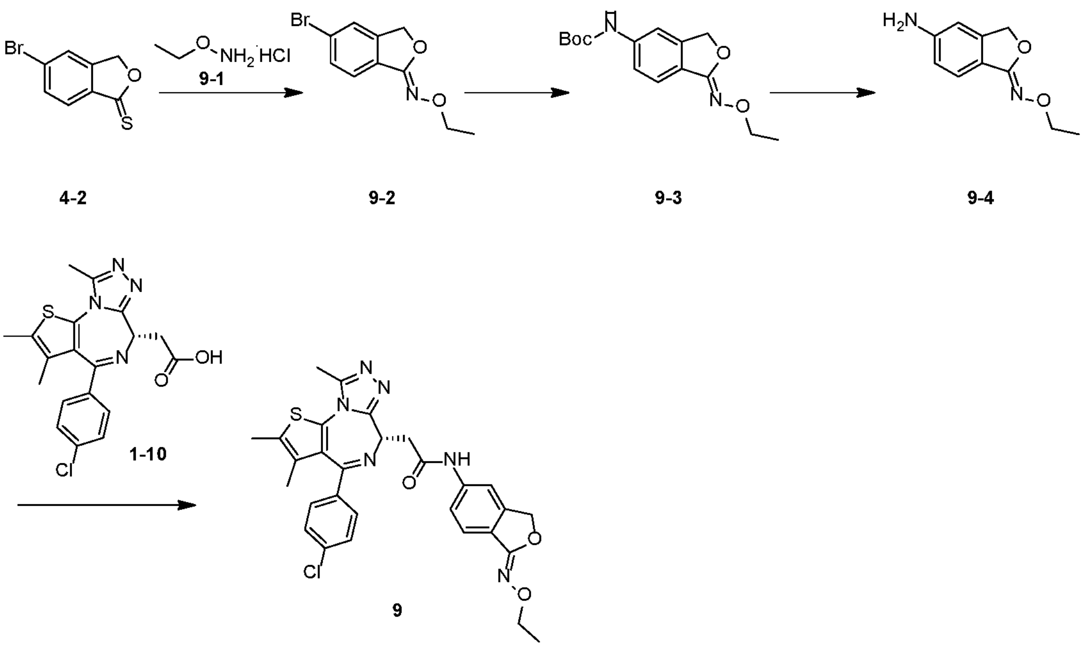

9-1

4-2 9-2 9-3 9-4

1-10

9

Example 9

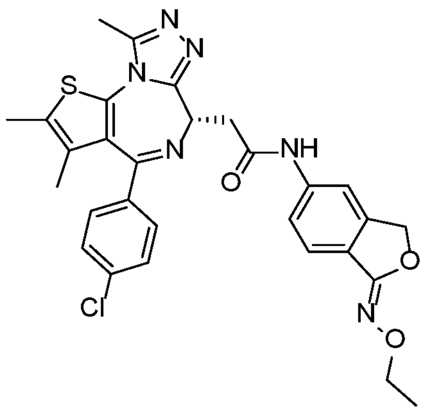

Synthesis of Compound 9-2

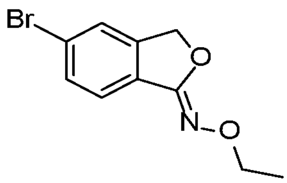

Compound 9-1(1.00g, 10.25mmol, 2.35eq) and NaOAc(895.16mg, 10.91mmol, 2.5eq) were added to a solution of Compound 4-2(1g, 4.37mmol, 1eq) in EtOH(10mL), and the reaction was stirred at 90 °C for 20 hours. LCMS showed that the starting materials were completely consumed and the target product was produced. The reaction was stopped. The reaction solution was cooled to room temperature and concentrated. The crude product obtained was dissolved in dichloromethane (50mL), and washed with water (50mL*2). The organic phase was dried over anhydrous sodium sulfate, and filtered. The filtrate was concentrated to afford the product. The obtained crude product was purified by column chromatography to afford Compound 9-2.

$^1$HNMR(400MHz, CDCl3)$\delta$=7.52-7.57(m, 3H), 5.44(s, 2H), 4.19(q, J=7.2Hz, 2H), 1.38(t, J=7.2Hz, 3H). LCMS(ESI)m/z: 255.5, 257.5(M+1, M+3).

Synthesis of Compound 9-3

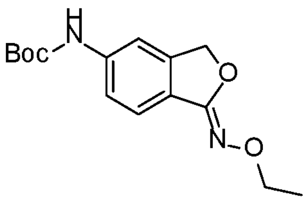

To a solution of Compound 9-2(200mg, 780.96$\mu$mol, 1eq) and tert-butyl carbamate (112.28mg, 958.46$\mu$mol, 1.23eq) in anhydrous 1,4-dioxane (5mL) were successively added 2-dicyclohexylphosphino-2',4',6'-triisopropylbiphenyl (44.21mg, 92.74$\mu$mol, 1.19e-1 eq), $Cs_2CO_3$(525.93mg, 1.61mmol, 2.07eq) and $Pd(dba)_2$(44.21mg, 76.89$\mu$mol, 9.85e-2 eq). The system was purged three times with nitrogen, and then the mixture was reacted in a microwave synthesizer at 120 °C for 0.5 h. LCMS showed that the starting materials were completely consumed and the target product was produced. The reaction was stopped. The reaction solution was diluted with ethyl acetate (10mL), washed successively with water (10mL) and saturated saline (10mL), dried over anhydrous sodium sulfate, and filtered. The filtrate was concentrated under reduced pressure to dryness. The residue was purified by column chromatography to afford Compound 9-3. LCMS(ESI)m/z: 292.9(M+1).

Synthesis of Compound 9-4

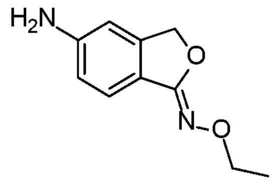

TFA(385.00mg, 3.38mmol, 250μL, 9.87eq) was added to a solution of Compound 9-3(0.1g, 342.08μmol, 1eq) in dichloromethane (1mL). The reaction was stirred at room temperature (25 °C) for 1 hour. LCMS showed that the starting materials were completely consumed and the target product was produced. The reaction was stopped. The reaction solution was diluted with dichloromethane (10mL) and neutralized with a saturated aqueous sodium bicarbonate solution to a pH of 7. The separated organic phase was washed with saturated saline (10mL), dried over anhydrous sodium sulfate, and filtered. The filtrate was concentrated to dryness under reduced pressure to afford Compound 9-4. LCMS(ESI)m/z: 192.7(M+1).

Synthesis of Compound 9

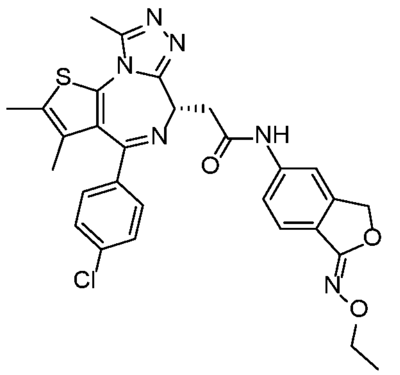

To a solution of Compound 9-4(35mg, 182.09μmol, 1eq), Compound 1-10(90mg, 224.51μmol, 1.23eq) and DMAP(2.22mg, 18.21μmol, 0.1eq) in anhydrous dichloromethane (2mL) were successively added DIPEA(74.20mg, 574.11μmol, 100μL, 3.15eq) and $T_3P$(181.90mg, 285.84μmol, 170μL, 50% purity, 1.57eq). The reaction was stirred at room temperature (25 °C) for 2 hours. LCMS showed that the starting materials were completely consumed and the target product was produced. The reaction was stopped. The reaction solution was diluted with dichloromethane (10mL) and washed with water (10mL). The organic phase was dried over anhydrous sodium sulfate and filtered. The filtrate was concentrated to dryness under reduced pressure. The resulting crude product was purified by preparative HPLC (basic) to afford Compound 9.

$^1$HNMR(400MHz, CDC13) δ=9.61(s, 1H), 7.83(s, 1H), 7.56(d, J=8.4Hz, 1H), 7.31-7.42(m, 5H), 5.36(s, 2H), 4.62-4.65 (m, 1H), 4.15(q, J=7.2Hz, 2H), 3.81-3.84(m, 1H), 3.50-3.54(m, 1H), 2.69(s, 3H), 2.41(s, 3H), 1.69(s, 3H), 1.35(t, J=7.2Hz, 2H). LCMS(ESI)m/z: 575.3(M+1).

Scheme 10

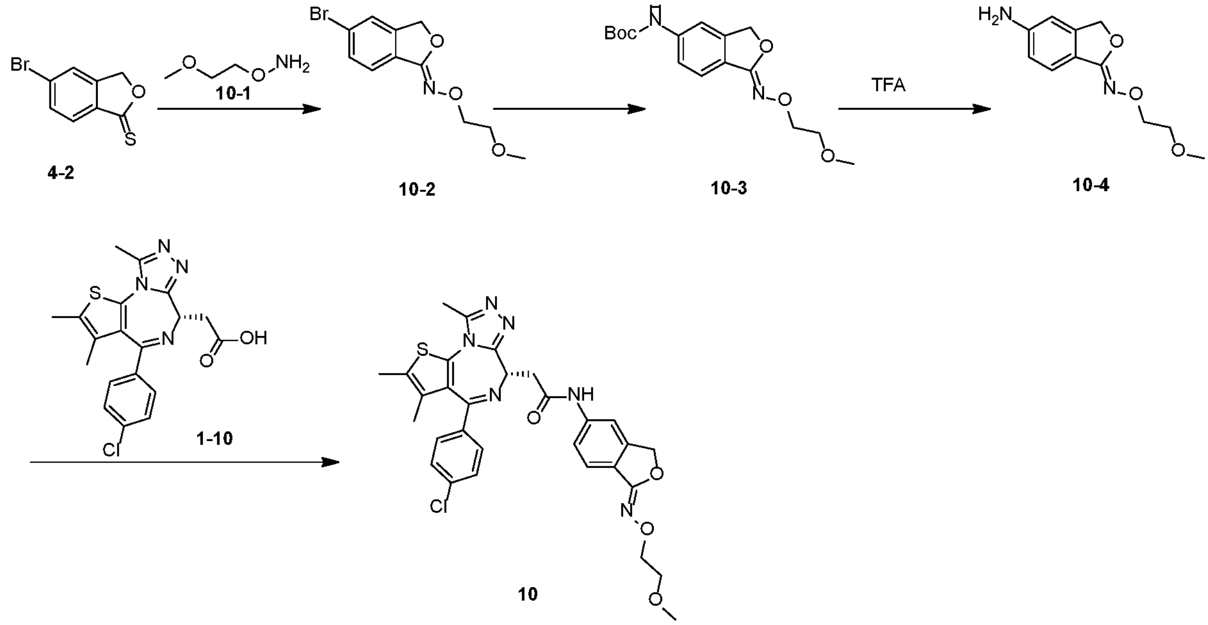


Example 10

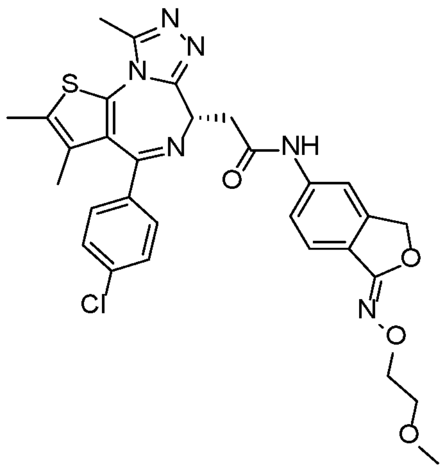

Synthesis of Compound 10-2

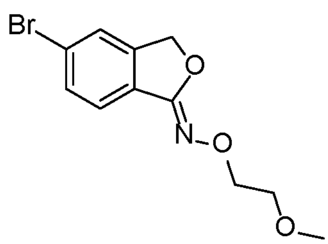

Compound 10-1(200.00mg, 2.20mmol, 2.51eq) and NaOAc(71mg, 865.50μmol, 9.91e-1 eq) were added to a solution of Compound 4-2(0.2g, 873.01μmol, 1eq) in EtOH(2mL) and the reaction was stirred at 90 °C for 20 hours. LCMS showed that the starting materials were completely consumed and the target product was produced. The reaction was stopped. The reaction solution was cooled to room temperature and concentrated to afford a crude product. The

crude product was dissolved in dichloromethane (10mL) and washed with water (5mL*2). The organic phase was dried over anhydrous sodium sulfate, and filtered. The filtrate was concentrated to afford a crude product. The crude product was purified by column chromatography to afford Compound 10-2. LCMS(ESI)m/z: 285.7, 287.7(M+1, M+3).

Synthesis of Compound 10-3

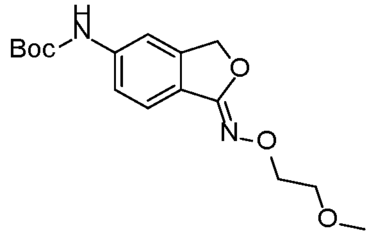

To a solution of Compound 10-2(80mg, 279.60μmol, 1eq) and tert-butyl carbamate (60mg, 512.18μmol, 1.83eq) in anhydrous 1,4-dioxane (1mL) were successively added 2-dicyclohexylphosphino-2',4',6'-triisopropylbiphenyl (16.00mg, 33.56μmol, 0.12eq), $Cs_2CO_3$(200.00mg, 613.84μmol, 2.20eq) and $Pd(dba)_2$(16.00mg, 27.83μmol, 9.95e-2 eq). The system was purged three times with nitrogen, and then the mixture was reacted in a microwave synthesizer at 120 °C for 0.5 h. TLC showed that the starting materials were almost completely consumed and the product was mainly produced. The reaction was stopped. The reaction solution was directly filtered, and the filtrate was concentrated under reduced pressure to dryness. The residue was purified by preparative TLC to afford compound 10-3. LCMS(ESI)m/z: 323.1(M+1).

Synthesis of Compound 10-4

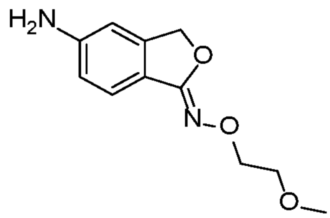

TFA(308.00mg, 2.70mmol, 200μL, 10.88eq) was added to a solution of Compound 10-3(80mg, 248.17μmol, 1eq) in dichloromethane (1mL), and the reaction was stirred at room temperature (20 °C) for 1 hour. LCMS showed that the starting materials were completely consumed and the target product was produced. The reaction was stopped. The reaction solution was diluted with dichloromethane (10mL) and neutralized with a saturated aqueous sodium bicarbonate solution to a pH of 7. The separated organic phase was washed with saturated saline (10mL), dried over anhydrous sodium sulfate, and filtered. The filtrate was concentrated under reduced pressure to dryness to afford Compound 10-4. LCMS(ESI)m/z: 222.6(M+1).

Synthesis of Compound 10

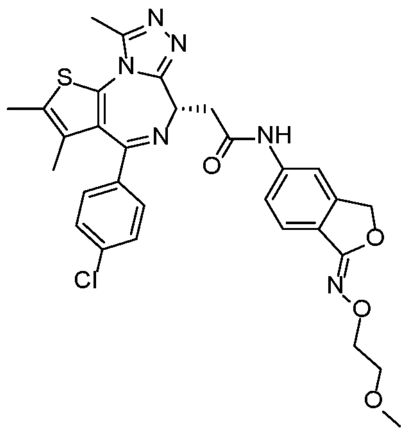

To a solution of Compound 10-4(50mg, 224.98μmol, 1eq), Compound 1-10(130mg, 324.29μmol, 1.44eq) and DMAP(3mg, 24.56μmol, 1.09e-1 eq) in anhydrous dichloromethane (2mL) were successively added DIPEA(111.30mg, 861.19μmol, 150μL, 3.83eq) and $T_3P$(267.50mg, 420.36μmol, 250μL, 50% purity, 1.87eq). The reaction was stirred at room temperature (19 °C) for 2 hours. LCMS showed that the starting materials were completely consumed and the target product was produced. The reaction was stopped. The reaction solution was diluted with dichloromethane (10mL) and washed with water (10mL). The organic phase was dried over anhydrous sodium sulfate and filtered. The filtrate was concentrated to dryness under reduced pressure. The resulting crude product was purified by preparative HPLC (basic) to afford Compound 10.

$^1$HNMR(400MHz, CDC13)$\delta$=9.87(s, 1H), 7.80(s, 1H), 7.52-7.50(m, 1H), 7.41-7.36(m, 3H), 7.32-7.26(m, 2H), 5.32(s, 2H), 4.70-4.67(m, 1H), 4.25-4.22(m, 2H), 3.87-3.84(m, 1H),, 3.70-3.73(m, 2H), 3.58-3.59(m, 1H), 3.40(s, 3H), 2.69(s, 3H), 2.41(s, 3H), 1.68(s, 3H). LCMS(ESI)m/z: 605.3(M+1).

Scheme 11

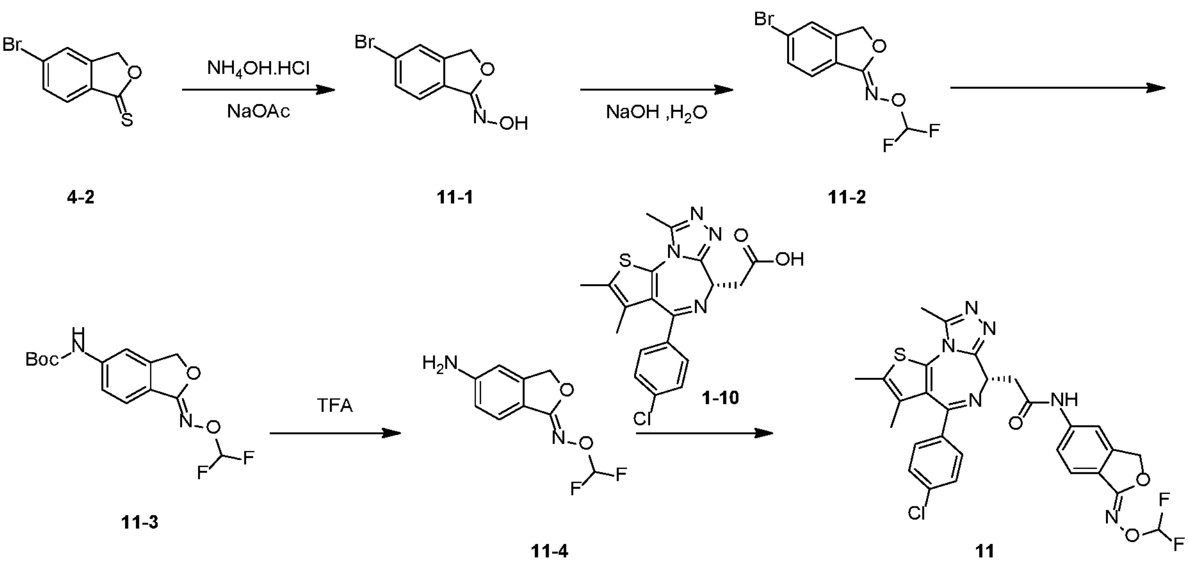


Example 11

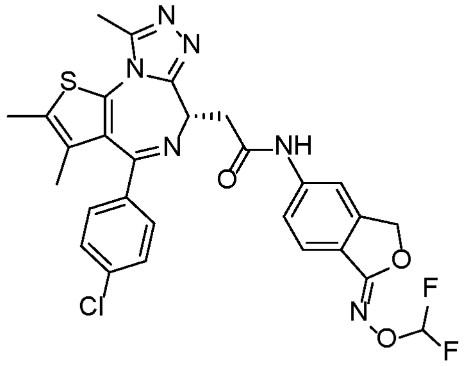

Synthesis of Compound 111

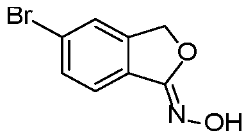

To a reaction flask were added EtOH(5mL), Compound 4-2(500mg, 2.18mmol, leq), $NH_2OH.HCl$(454.99mg, 6.55mmol, 3eq) and NaOAc(537.10mg, 6.55mmol, 3eq). The reaction was stirred at room temperature (20 °C) for 1 hour. LCMS showed that the starting materials were completely consumed and the target product was produced. The system was filtered, and the filter cake was washed with 10mL of ethanol. The filtrate was concentrated to dryness, and the residue was then purified by column chromatography to afford Compound 11-1. LCMS(ESI)m/z: 227.9, 229.9(M+1, M+3).

Synthesis of Compound 11-2

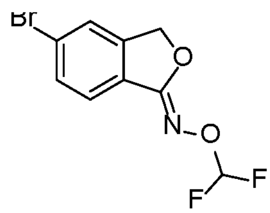

To a reaction flask were added toluene (35mL), $H_2O$(11mL), and NaOH(4.43g, 110.83mmol, 24.07eq), followed by Compound 11-1(1.05g, 4.60mmol, 1eq) and TBAI(350.00mg, 947.57μmol, 2.06e-1 eq). The mixture was bubbled with chlorodifluoromethane (398.14mg, 4.60mmol, 1 eq) at room temperature (20 °C) for 1 hour. Under chlorodifluoromethane (398.14mg, 4.60mmol, 1eq) balloon of 15psi, the reaction was then heated in an oil bath at 80 °C for 9 hours. LCMS showed that the starting materials were remained and the target product was produced. To the reaction system was added 200mL of water. The mixture was extracted twice respectively with 50mL of ethyl acetate. The organic phases were combined and washed once more with 30mL of water and once more with 30mL of saturated saline. The organic phase was then dried over anhydrous sodium sulfate, and filtered. The filtrate was concentrated to dryness. The residue was purified by column chromatography to afford Compound 11-2. LCMS(ESI)m/z: 277.9, 279.9(M+1, M+3).

Synthesis of Compound 11-3

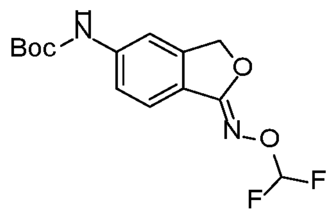

To a reaction flask were added toluene (2mL), Compound 11-2(140.00mg, 503.51μmol, leq), tert-butyl carbamate (182.26mg, 1.56mmol, 3.09eq), $Cs_2CO_3$(411.60mg, 1.26mmol, 2.51eq), Xantphos(30.80mg, 53.23μmol, 1.06e-1 eq) and $Pd_2(dba)_3$(47.60mg, 51.98μmol, 1.03e-1 eq). The system was purged three times with nitrogen, and then the mixture was heated to 120 °C and reacted for 18 hours. LCMS showed that the starting materials were completely consumed and the target product was produced. The reaction solution was concentrated to dryness under reduced pressure, and 20mL of ethyl acetate and 20mL of water were added. After extraction, the layers were separated. The organic phase was dried over anhydrous sodium sulfate, and filtered. The filtrate was concentrated to afford a crude product. The crude product was purified by column chromatography to afford Compound 11-3. LCMS(ESI)m/z: 315.1(M+1).

Synthesis of Compound 11-4

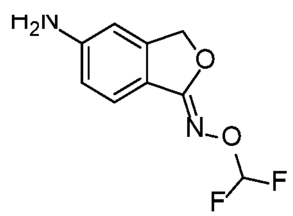

To a reaction flask were added dichloromethane (3mL), Compound 11-3(110.00mg, 350.00μmol, 1eq) and TFA(1.54g, 13.51mmol, 1mL, 38.59eq). The mixture was reacted at 15 °C for 1 hour. TLC showed disappearance of starting materials and appearance of a new product. To the reaction system were added 20mL of saturated sodium bicarbonate solution, and 10mL of dichloromethane. The layers were separated. The organic phase was dried over anhydrous sodium sulfate, and filtered. The filtrate was concentrated to afford Compound 11-4. LCMS(ESI)m/z: 215.0(M+1).

Synthesis of Compound 11

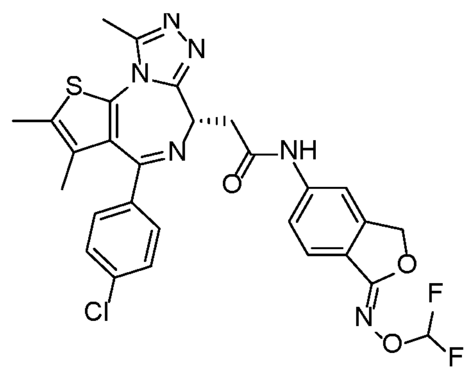

To a reaction flask were added dichloromethane (2mL), Compound 1-10(70mg, 174.62μmol, 1eq) and Compound 11-4(41.14mg, 192.08μmol, 1.1eq), followed by DMAP(2.1mg, 17.19μmol, 9.84e-2 eq). The system was purged three times with nitrogen, and then DIPEA(32mg, 247.60μmol, 43.13μL, 1.42eq) and $T_3P$(167mg, 262.43μmol, 156.07μL, 50% solution in ethyl acetate, 1.5eq) were added to the reaction. The mixture was reacted at room temperature

(20 °C) for 2 hours. LCMS showed disappearance of the starting materials and production of the target product. To the reaction system was added 20mL of water, followed by 20mL of dichloromethane. The layers were separated. The aqueous phase was further extracted with 10mL of dichloromethane. The organic phases were combined, dried over anhydrous sodium sulfate, and filtrated. The filtrate was concentrated to afford a crude product. The crude product was purified by preparative HPLC (basic) to afford Compound 11.

$^1$HNMR(400MHz, CDCl3)δ=10.33(s, 1H), 7.80(s, 1H), 7.49-7.47(m, 1H), 7.41-7.39(m, 3H), 7.31-7.29(m, 1H), 6.82-6.31(m, 1H), 5.33(s, 2H), 4.77-4.74(m, 1H), 3.99-3.98(m, 1H), 3.59-3.54(m, 1H), 2.72(s, 3H), 2.44(s, 3H), 1.71(s, 3H). LCMS(ESI)m/z: 597.2(M+1).

Scheme 12

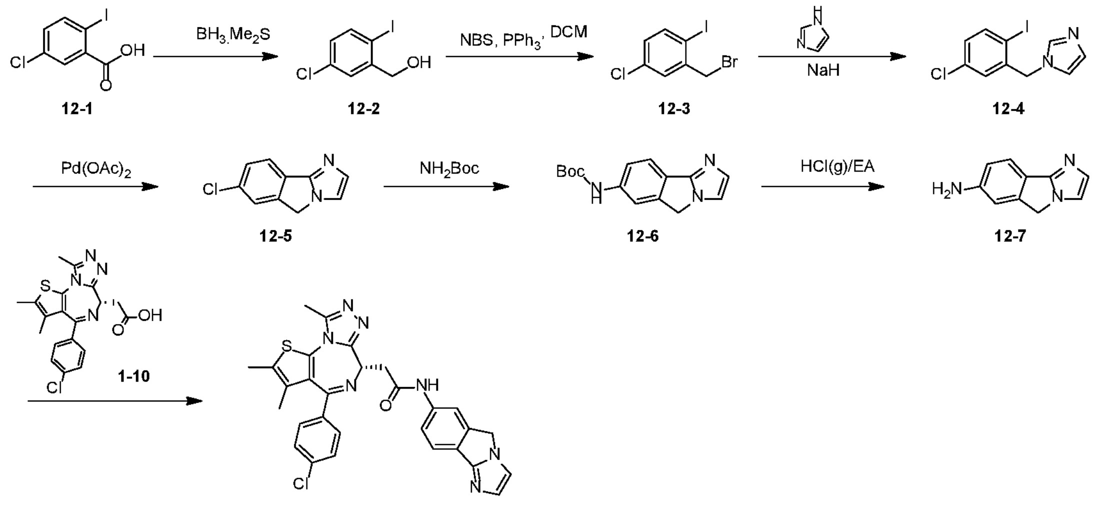


12

Example 12

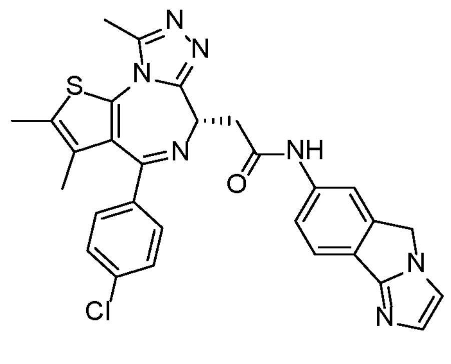

Synthesis of Compound 12-2

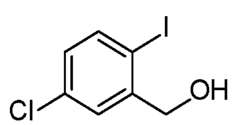

To a 100mL three-necked flask were added Compound 12-1(5g, 17.70mmol, 1eq) and THF(50mL). The reaction was cooled to 0 °C. Borane dimethyl sulfide (10M, 3.54mL, 2eq) was added to the reaction at 0-5 °C. The reaction was slowly warmed to room temperature (20 °C) and stirred for 12 hours. The reaction was then heated to 50 °C and stirred for 6 hours. TLC showed that the starting materials were completely consumed and one main point appeared. The

reaction solution was cooled to 0 to 5 °C, and anhydrous methanol (50mL) was slowly added to the solution at this temperature with bubbles formed. After the dropwise addition, the mixture was heated to 80 °C in an oil bath, and refluxed for 1 hour. The reaction was fully quenched, and the mixture was concentrated. The residue was purified by column chromatography to afford Compound 12-2.

$^1$HNMR(400MHz, CDCl3)δ=7.73(d, J=8.4Hz, 1H), 7.49(d, J=2.4Hz, 1H), 7.02(d, J=8.4, 2.4Hz, 1H), 4.65(d, J=6.0Hz, 2H), 2.02(t, J=6.0Hz, 1H).

Synthesis of Compound 12-3

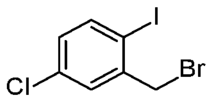

To a reaction flask were added dichloromethane (75mL), NBS(12.43g, 69.84mmol, 2.5eq), and $PPh_3$(14.65g, 55.87mmol, 2eq) at 0 °C. Compound 12-2(7.5g, 27.94mmol, 1eq) was added to the reaction at 0 °C. The reaction was stirred at room temperature (20 °C) for 16 hours. LCMS and TLC showed that the starting material points disappeared and a new point appeared. 100mL of water was added to the system. The layers were separated. The organic phase was washed with 100mL of saturated saline, dried over anhydrous sodium sulfate, and filtered. The filtrate was concentrated under reduced pressure. The residue was purified by column chromatography to afford Compound 12-3.

$^1$HNMR(400MHz, CDCl3)δ=7.77(d, J=8.4Hz, 1H), 7.47(d, J=2.6Hz, 1H), 6.99(dd, J=2.5, 8.4Hz, 1H), 4.53(s, 2H).

Synthesis of Compound 12-4

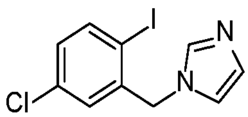

To a reaction flask was added Compound 12-3(5g, 15.09mmol, 1eq) in THF(50mL), and the mixture was cooled to 0 °C. NaH(663.90mg, 16.60mmol, 60% purity, 1.1eq) was added to the reaction in batches at 0-5 °C and the mixture was reacted at 0-5 °C for 0.5 h. Imidazole (1.08g, 15.84mmol, 1.05eq) was then added to the reaction. The reaction was slowly warmed to room temperature (20 °C) and further stirred for 12 hours. TLC showed that the starting material points disappeared and a main point appeared. Saturated ammonium chloride (100mL) was added to the reaction solution. The layers were separated. The organic phase was extracted with ethyl acetate (100mL*2). The organic phases were combined, washed with saturated saline (50mL), dried over anhydrous sodium sulfate, and filtered. The filtrate was concentrated and the residue was purified by column chromatography to afford Compound 12-4.

$^1$HNMR(400MHz, CDCl3)δ=7.81(d, J=8.4Hz, 1H), 7.59(s, 1H), 7.16(s, 1H), 7.05(dd, J=8.4, 2.4Hz, 1H), 6.95(s, 1H), 6.79(s, 1H), 5.13(s, 2H). LCMS(ESI)m/z: 318.9(M+1).

Synthesis of Compound 12-5

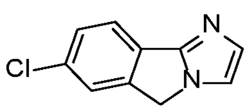

To a microwave tube were added Compound 12-4(1g, 3.14mmol, 1eq) and DMF(12mL). $Pd(OAc)_2$(70.48mg, 313.93μmol, 0.1eq), tris(2-methylphenyl)phosphine (286.65mg, 941.80μmol, 0.3eq) and AcOK(616.19mg, 6.28mmol, 2eq) were successively added to the reaction. The reaction was heated with microwave at 150 °C for 1 hour (0bar). LCMS showed that the starting materials were completely consumed and the target product was produced. The reaction solution was cooled to room temperature. Water (100mL) was added to the reaction, and the mixture was stirred for 2

minutes. The mixture was extracted with ethyl acetate (100mL*3). The combined organic phase was washed with saturated saline (100mL), dried over anhydrous sodium sulfate, and filtered. The filtrate was concentrated under reduced pressure to afford a crude product. The crude product was purified by column chromatography to afford Compound 12-5.

$^1$HNMR(400MHz, CDCl3)δ=7.74(s, 1H), 7.49(d, J=8.0Hz, 1H), 7.40-7.36(m, 2H), 7.20(s, 1H), 5.02(s, 2H). LCMS(ESI)m/z: 191.0(M+1).

Synthesis of Compound 12-6

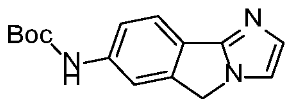

To a reaction flask were added degassed 1,4-dioxane (8.8mL) and Compound 12-5(880mg, 4.62mmol, 1eq). Tert-butyl carbamate (1.62g, 13.85mmol, 3eq), $Cs_2CO_3$(3.76g, 11.54mmol, 2.5eq), 2-dicyclohexylphosphino-2',4',6'-triisopropylbiphenyl (220.07mg, 461.63μmol, 0.1eq) and $Pd(dba)_2$(530.88mg, 923.26μmol, 0.2eq) were successively added to the reaction. The reaction was heated to 105 °C and stirred for 12 hours. LCMS showed that the starting materials were completely consumed and the target product was produced. The reaction solution was cooled to room temperature and concentrated under reduced pressure. Ethyl acetate (20mL) and $H_2O$(20mL) were added, and insolubles were filtered off through a pad of celite. The layers were separated, and the aqueous phase was extracted with ethyl acetate (20mL*2). The combined organic phase was washed with saturated saline (10mL), dried over anhydrous sodium sulfate, and filtered. The filtrate was concentrated to afford Compound 12-6. LCMS(ESI)m/z: 272.1(M+1).

Synthesis of Compound 12-7

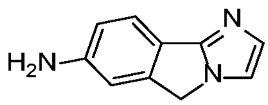

To a reaction flask were added Compound 12-6(0.9g, 3.32mmol, 1eq) and ethyl acetate (0.5mL). After the starting materials were dissolved, the system was cooled to 0-5 °C. HCl (gas)/ethyl acetate (4M, 14.93mL, 18eq) was added to the system. After the addition, the reaction was slowly warmed to room temperature (20 °C) and further stirred for 12 hours. TLC showed that the starting materials were completely consumed and the target product was produced. The reaction solution was washed with water (20mL*2). The combined aqueous phase was cooled to 0-5 °C, and saturated aqueous solution of sodium bicarbonate was added to adjust pH to ~8. The aqueous phase was extracted with ethyl acetate (30mL*3) and the combined organic phase was washed with saturated saline (10mL). The organic phase was dried over anhydrous sodium sulfate, and filtered. The filtrate was concentrated. The residue was purified by column chromatography to afford Compound 12-7.

$^1$HNMR(400MHz, CDCl3)δ=7.65(s, 1H), 7.35(d, J=8.0Hz, 1H), 7.02(s, 1H), 6.73(s, 1H), 6.70-6.68(m, 1H), 4.92(s, 2H), 3.76(brs, 2H). LCMS(ESI)m/z: 172.1(M+1).

Synthesis of Compound 12

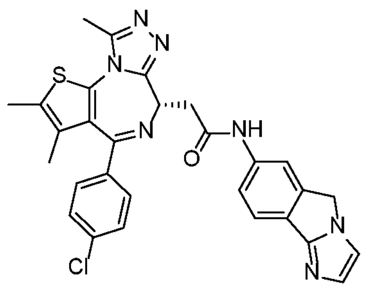

To a reaction flask were added Compound 1-10(18.45mg, 107.76μmol, 1.08eq) and dichloromethane (2mL). Compound 12-7(40mg, 99.78μmol, 1eq) and DMAP(1.22mg, 9.98μmol, 0.1eq) were then added to the reaction. The system was purged 3 times with nitrogen. DIPEA(17.80mg, 137.70μmol, 23.98μL, 1.38eq) and $T_3P$(90.16mg, 141.69μmol, 84.27μL, 50% purity, 1.42eq) were then added to the reaction. The reaction was stirred at room temperature (20 °C) for 2 hours. LCMS showed that the starting materials were completely consumed and the target product was produced. To the reaction solution were added dichloromethane (10mL) and $H_2O$(10mL), and the mixture was stirred for 2min. The layers were separated, and the aqueous phase was extracted with dichloromethane (5mL*2). The combined organic phase was washed with saturated saline (5mL), dried over anhydrous sodium sulfate, and filtered. The filtrate was concentrated to afford a crude product. The crude product was purified by preparative HPLC (basic) to afford Compound 12.

$^1$HNMR(400MHz, CDCl3)δ=9.45(s, 1H), 7.85(s, 1H), 7.63(s, 1H), 7.44-7.40(m, 3H), 7.35-7.31(m, 3H), 7.10(s, 1H), 4.92-4.79(m, 2H), 4.70-4.66(m, 1H), 3.93-3.87(m, 1H), 3.53-3.49(m, 1H), 2.72(s, 3H), 2.43(s, 3H), 1.71(s, 3H). LCMS(ESI)m/z: 554.2(M+1).

Scheme 13

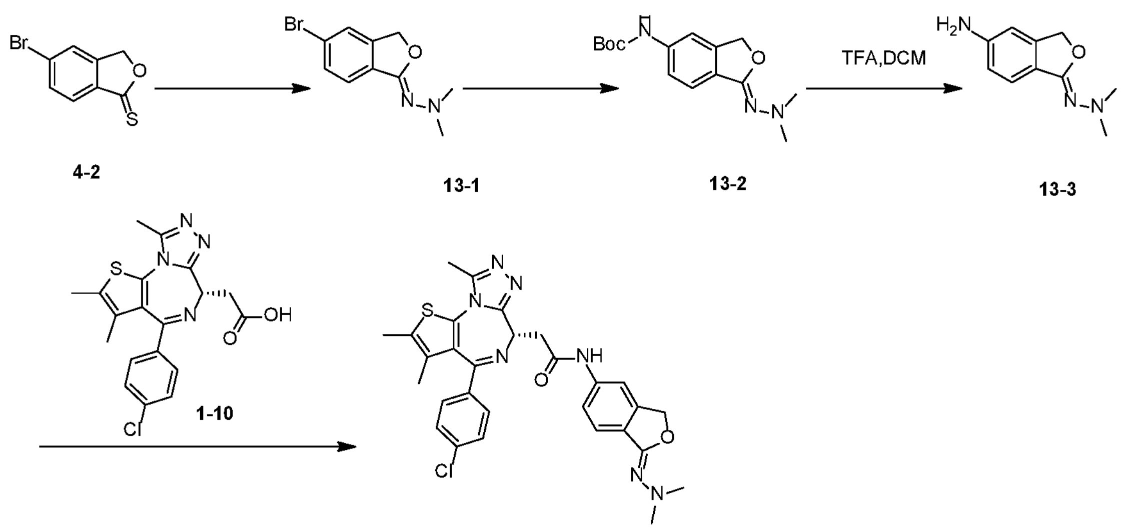


4-2 13-1 13-2 13-3

1-10

13

Example 13

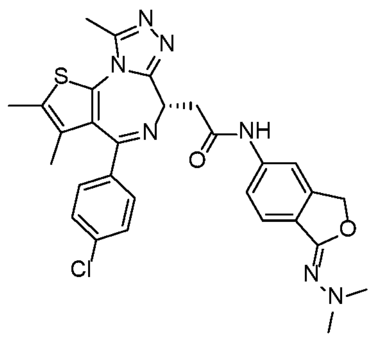

Synthesis of Compound 13-1

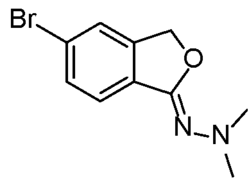

1,1-dimethylhydrazine hydrochloride (720.00mg, 7.46mmol, 909.09μL, 1.42eq) and NaOAc(1.3g, 15.85mmol, 3.03eq) were added to a solution of Compound 4-2(3.0g, 5.24mmol, 1eq) in EtOH(30mL), and the reaction was stirred at 90 °C for 20 hours. LCMS showed complete consumption of the starting materials and production of the product (23.11%). The reaction was stopped. The reaction solution was cooled to room temperature, and water (20mL) and dichloromethane (20mL) were added. The mixture was stirred at room temperature for 5 minutes, and then allowed to stand for layer separation. The organic phase was separated, washed with saturated saline (20mL), dried over anhydrous sodium sulfate, and filtered. The filtrate was concentrated under reduced pressure to dryness. The obtained crude product was purified by column chromatography to afford Compound 13-1. LCMS(ESI)m/z: 254.5, 256.5(M+1, M+3).

Synthesis of Compound 13-2

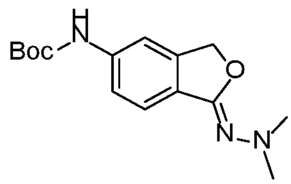

To a solution of Compound 13-1(85mg, 333.19μmol, 1eq) and tert-butyl carbamate (80mg, 682.91μmol, 2.05eq) in anhydrous 1,4-dioxane (2mL) were successively added 2-dicyclohexylphosphino-2',4',6'-triisopropylbiphenyl (17.00mg, 35.66μmol, 1.07e-1 eq), $Cs_2CO_3$(600.00mg, 1.84mmol, 5.53eq) and $Pd(dba)_2$(17.00mg, 29.56μmol, 8.87e-2 eq). The system was purged three times with nitrogen, and then the mixture was reacted in a microwave synthesizer at 120 °C for 1 hour. LCMS showed that the starting materials were completely consumed and the target product was produced. The reaction solution was purified by preparative TLC to afford Compound 13-2. LCMS(ESI)m/z: 291.9(M+1).

Synthesis of Compound 13-3

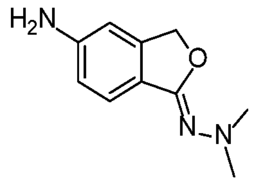

TFA(308.00mg, 2.70mmol, 200μL, 11.24eq) was added to a solution of Compound 13-2(70mg, 240.26μmol, 1eq) in dichloromethane (1mL), and the reaction was stirred at room temperature (20 °C) for 1 hour. LCMS showed that the starting materials were completely consumed and the target product was produced. The reaction solution was diluted with dichloromethane (10mL) and then neutralized with a saturated aqueous sodium bicarbonate solution to a pH of 7. The separated organic phase was washed with saturated saline (10mL), dried over anhydrous sodium sulfate, and filtered. The filtrate was concentrated to dryness under reduced pressure to afford Compound 13-3. LCMS(ESI)m/z: 191.9(M+1).

Synthesis of Compound 13

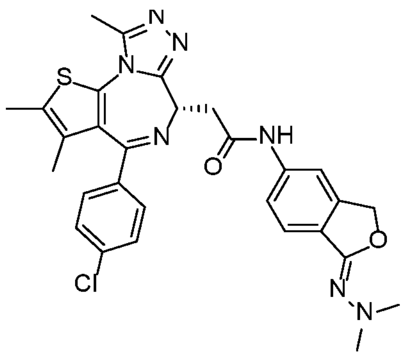

To a solution of Compound 13-3(25mg, 130.73μmol, leq), Compound 1-10(80mg, 199.56μmol, 1.53eq) and DMAP(2mg, 16.37μmol, 1.25e-1 eq) in anhydrous dichloromethane (2mL) were successively added DIPEA(59.36mg, 459.30μmol, 80μL, 3.51eq) and $T_3P$(149.80mg, 235.40μmol, 140μL, 50% solution in ethyl acetate, 1.80eq). The reaction was stirred at room temperature (19 °C) for 2 hours. LCMS showed that the starting materials were completely consumed and the target product was produced. The reaction solution was diluted with dichloromethane (10mL) and washed with water (10mL). The organic phase was dried over anhydrous sodium sulfate and filtered. The filtrate was concentrated to dryness under reduced pressure. The resulting crude product was purified by preparative HPLC (basic) to afford Compound 13.

$^1$HNMR(400MHz, CDCl3)δ=9.61(s, 1H), 7.83(s, 1H), 7.64-7.62(m, 1H), 7.42-7.40(m, 2H), 7.35-7.33(m, 3H), 5.32(s, 2H), 4.66-4.63(m, 1H), 3.87-3.81(m, 1H), 3.55-3.51(m, 1H), 2.69(s, 3H), 2.68(s, 6H), 2.41(s, 3H), 1.69(s, 3H). LCMS(ESI)m/z: 573.9(M+1).

**Biological Assay**

**Assay Example 1: Assay of BRD4 biochemical activity of the compounds of the present disclosure**

1. Assay Preparation

Test 1: $IC_{50}$ characterization of the compounds at 10 concentrations.

The compounds were evaluated for $IC_{50}$ against two BRD (BRD4-1, BRD4-2) at 10 concentrations, single well.

2. Test condition

Buffer composition for BRD: 50mM HEPES-HCl, pH7.5, 100mM NaCl, 0.1%BSA, 0.05%CHAPS, and 1%DMSO.

Ligand: histone H4 peptide (1-21) K5/8/12/16Ac-Biotin

Assay: AlphaScreen binding assay (Ex/Em=680/520-620nm)

Assay procedures:

2.1 4XBRD was added into wells of a reaction plate, except for control wells without BRD, in which BRD was replaced with a buffer.
2.2 A solution of the compounds in 100% DMSO was added to the BRD mixture using Acoustic Technology (Echo550, nanoliter level). The mixture was centrifuged, shaken gently at room temperature, and pre-incubated for 30 minutes.
2.3 4X ligand was added, and the mixture was centrifuged and shaken.
2.4 The mixture was shaken gently and incubated at room temperature for 30 minutes.
2.5 4X donor beads were added in the dark, and the mixture was centrifuged and shaken.
2.6 4X acceptor beads were added in the dark, and the mixture was centrifuged and shaken gently in the dark for 60 minutes.
2.7 The Alpha assay was carried out using Enspire (Ex/Em=680/520-620nm)

Data analysis:

The signal value of the control well (DMSO) was defined as 100% enzyme activity, and the signal value of the background well (in the buffer, no BRD was added but ligand was added) was defined as 0% enzyme activity (or 100% inhibition).

The percent enzyme activity for each test solution was calculated by Mcrosoft Excel 2003 or 2007 software using the following equation:

Percent enzyme activity = {{[signal value]-[background signal value]}/{[signal value of DMSO control well]-[ background signal value]}} x 100

The four-parameter fitting was performed by GraphPadPrism4 software using the following formula to obtain an $IC_{50}$ fitting curve:

Y= minimum+(maximum-minimum)/(1+10^((LogIC50-X) * HillSplope))

Prism settings: 4-parameter dose-response curve (with variable slope), constrained; minimum =0, maximum = less than 120

The curve fitting was performed when the percentage enzyme activity corresponding to the compounds at the highest concentration was less than 65%.

3. Result

Test 1: $IC_{50}$ characterization of the compounds at 10 concentrations.

The compounds were tested for $IC_{50}$ against BRD4-1 and BRD4-2 at 10 concentrations, single well, initial concentration of 10μM, and 3-fold serial dilution.

$IC_{50}$ values (molar concentration) were summarized in the table below.

Summary Table: $IC_{50}$(M)

Table 1 $IC_{50}$ Results of BRD4 Assay

| Compound | BRD4 (BD1, BD2), $IC_{50}$(nM) |
|---|---|
| 1 | 48.8, 7.4 |
| 2 | 48.2, 6.23 |
| 3 | 27.0, 4.45 |
| 4 | 28.0, 6.6 |
| 5 | 31.2, 4.06 |

(continued)

| Compound | BRD4 (BD1, BD2), $IC_{50}$(nM) |
|---|---|
| 6 | 63.8, 15.1 |
| 7 | 28.7, 6.06 |
| 8 | 85.7, 15.0 |
| 9 | 43.6, 7.0 |
| 10 | 27.0, 3.56 |
| 11 | 72.2, 3.92 |
| 12 | 18.1, 1.99 |
| 13 | 13.1, 2.26 |

Conclusion: The compounds of the present disclosure all have significant activity of inhibiting BET bromdomain.

**Assay Example 2: Regulation of cell PD-L1 by the compounds of the present disclosure (1) Effect of the compounds on PD-L1 of MCF7 cells**

**Assay purpose:**

The downregulation of the compounds on PD-L1 gene was evaluated by detecting the effect of the compounds on PD-L1 of MCF7 cells through a qPCR test.

**Assay method:**

MCF7 cells were stimulated with 5 μM of the compounds and interferon y, respectively. The cells were cultured for 18 hours, and then the samples were collected and detected by qPCR method. The content of DMSO in the detection reaction was 0.1%.

**Reagents:**

Takara PrimeScript™ RT Master Mix Kit-RR036A;
Thermo Power SYBR™ Green PCR Master Mix Kit-4367659;
QIAGEN RNeasy Mini Kit-74106.

**Compound:**

The test compound was dissolved in 100% DMSO and diluted to 10 mM for subsequent use. Interferon γ was diluted with PBS to a final concentration of 100 ng/mL.

**Assay procedures:**

To cell samples were respectively added each compound and interferon γ to a final concentration of 5 μM and 100 ng/mL. After incubation with drugs for 18 hours, RNA was extracted from the cells using RNeasy kit and inverted to cDNA using Takara inversion kit. Gene primers and SYBR™ Green reagent were added to the cDNA to detect the relative content of the target gene by qPCR.

**Reaction detection:**

The relative abundance of the target gene was obtained by reading the plate with QuantStudio 7 instrument.
The assay results were shown in Figure 1.
**Assay conclusion:** The compounds of the present disclosure have significant down-regulation effect on PD-L1 gene expression.

**(2) Effect of the compounds on PD-L1 of MDA-MB-231 cells**

**Assay purpose:**

The downregulation of the compounds on PD-L1 gene was evaluated by detecting the effect of the compounds on PD-L1 of MDA-MB-231 cells through a qPCR test.

**Assay method:**

MDA-MB-231 cells were stimulated with each compound at 250 nM, and cultured for 18 hours. The samples were collected and detected by qPCR method. The content of DMSO in the detection reaction was 0.1%.

**Reagents:**

Takara PrimeScript™ RT Master Mix Kit-RR036A
Thermo Power SYBR™ Green PCR Master Mix Kit-4367659
QIAGEN RNeasy Mini Kit-74106.

**Compound:**

The test compound was dissolved in 100% DMSO and diluted to 10 mM for subsequent use.

**Assay procedures:**

Each compound was added to cell samples to a final concentration of 250 nM, respectively. After incubation with drugs for 18 hours, RNA was extracted from the cells using RNeasy kit and inverted to cDNA using Takara inversion kit. Gene primers and SYBR™ Green reagent were added to the cDNA to detect the relative content of the target gene by qPCR.

**Reaction detection:**

The relative abundance of the target gene was obtained by reading the plate with QuantStudio 7 instrument.
The assay results were shown in Figure 2.
**Assay conclusion:** The compounds of the present disclosure have efficient down-regulation effect on PD-L1 gene expression.

**Assay Example 3: Pharmacokinetic studies of the compounds of the present disclosure**

1. Abstract

1.1 Male CD-1 mice were used as test animals, and the LC/MS/MS method was used to determine the drug concentration in plasma of mice at different times after intravenous and intragastric administration of the test compounds. The pharmacokinetic behavior of the compounds of the present disclosure in mice was studied, and the pharmacokinetic characteristics of the compounds of the present disclosure were evaluated.

2. Assay scheme

2.1 Assay drugs: test compounds.
2.2 Assay animals: Eight healthy adult male CD-1 mice, which were divided into four groups (two for each group) according to the principle of similar body weight. The animals were purchased from Shanghai Sippe-Bk Lab Animal Co.,Ltd., with animal production license No. of SCXK (Shanghai) 2018-0006.
2.3 Drug preparation
A proper amount of samples were weighed, to which a solvent was added. The mixture was stirred with ultrasound to a clear state for intravenous administration.
A proper amount of samples were weighed, to which a solvent was added. The mixture was stirred with ultrasound to a clear state for intragastric administration.

2.4 Administration

Eight male CD-1 mice were divided into four groups. After one-night fasting, two groups were administered intravenously, and the remaining two groups were administered intragastrically.

3. Operation

After intravenous administration of the test compounds to male CD-1 mice, 30μL of blood was collected at 0.0833, 0.25, 0.5, 1, 2, 4, 8, and 24 hours, and placed in commercial tubes containing EDTA-$K_2$. After intragastric administration of the test compounds, 30μL of blood was collected at 0.25, 0.5, 1, 2, 4, 6, 8, and 24 hours, and placed in commercial tubes containing EDTA-$K_2$. The tubes were centrifuged at 3000g for 15 minutes to separate plasma and stored at -60 °C. Animals were fed 2 hours after the administration.

The LC/MS/MS method was used to determine the content of the test compounds in the plasma of mice after intravenous and intragastric administration. The linear range of the method was 2.00-6000 nmol/L; The plasma samples were analyzed after protein precipitation with acetonitrile.

4. Pharmacokinetic parameter results

Table 2 Summary of Pharmacokinetic Parameter Data

| Mode of administrati on | Dose adminis tered | Drug concen tration in blood | Peak arrival time | Half life period | Appare nt volume of distribu tion | Clearanc e rate | Area under curve (0-t) | Area under curve (0-inf) | bioavailabi lity |
|---|---|---|---|---|---|---|---|---|---|
| | | Cmax( nM) | Tmax( h) | T1/2 (h ) | Vdss(L /kg) | Cl(mL/m in/kg) | $AUC_{0\text{-}last}$(n M.h) | $AUC_{0\text{-}inf}$(n M.h) | F (%) |
| Compound 4 is administered intravenousl y | 1mg/kg | - | - | 2.05 | 1.84 | 10.2 | 2715 | 2910 | - |
| Compound 4 is administered intragastrica lly | 2mg/kg | 262 | 4.00 | ND | - | - | 1606 | ND | 29.6 |
| Compound 11 is administered intravenousl y | 1mg/kg | - | - | 2.16 | 2.21 | 11.5 | 2260 | 2439 | - |
| Compound 11 is administered intragastrica lly | 2mg/kg | 115 | 7.00 | ND | - | - | 1311 | ND | 29.0 |
| "--": none;<br>ND: not detected. | | | | | | | | | |

Assay conclusion: The compounds of the present disclosure have short half-life period, extensive distribution outside plasma, and moderate bioavailability.

**Assay Example 4. Study on Anti-tumor Efficacy in Subject**

**(1) In vivo efficacy study of the compounds of the present disclosure in an animal transplanted tumor model of colon cancer cells in MC38 mice**

1. Assay design

Table 3 Animal grouping and administering scheme of in vivo efficacy assay

| Group | Number of animals | Compound therapy | Dose (mg/kg) | Administering volume parameters (1/g) | Route of administration | Administering frequency |
|---|---|---|---|---|---|---|
| 1 | 8 | Vehicle | - | 10 | p.o. | BID |
| 2 | 8 | Compound 4 | 20 | 10 | p.o. | BID |
| 3 | 8 | Compound 4 | 50 | 10 | p.o. | BID |
| 4 | 8 | Compound 11 | 20 | 10 | p.o. | BID |
| 5 | 8 | Compound 11 | 50 | 10 | p.o. | BID |
| *p.o: oral; BID: twice a day. | | | | | | |

2. Assay materials

2.1 Assay animals

Species: mice.

Strain: C57BL/6 mice.

Week age and body weight: 6-8 weeks old.

Gender: female.

Supplier: Shanghai slack Laboratory Animal Co., Ltd.

3. Assay method and steps

3.1 Cell culture

Mouse colon cancer MC38 cells (Art. No. HYC3401) were cultured in vitro in single layer under the conditions of DMEM containing 10% fetal bovine serum and 37 °C 5% $CO_2$ incubator. Trypsin-EDTA was used for routine digestion and passage. When the cells were in exponential growth phase and the saturation was 80%-90%, the cells were collected and counted.

3.2 Tumor cell inoculation

Cells were re-suspended in DPBS at a density of $2\times10^5$ cells/mL. 0.1 mL DPBS (containing $2\times10^5$ MC38 cells) was subcutaneously inoculated into the right back of each mouse. When average tumor volume reached ~63 $mm^3$, mice

were randomly grouped and administered according to tumor volume.

3.3 Tumor measurement and assay indicators

Tumor diameter was measured with vernier caliper three times a week. The calculation formula of tumor volume was as follows: $V = 0.5 \times a \times b^2$, wherein a and b indicated the long and short diameters of a tumor, respectively.

The anti-tumor effect of the compounds was evaluated using TGI(%) or relative tumor proliferation rate T/C(%). Relative tumor proliferation rate T/C (%) = $T_{RTV}$;/$C_{RTV}$ $\times$ 100 ($T_{RTV}$: average RTV for treatment group; $C_{RTV}$: average RTV for negative control group). According to the results of tumor measurement, relative tumor volume (RTV) was calculated using the formula: RTV = Vt/Vo, wherein Vo is the tumor volume measured when grouped and administered (i.e., D0), Vt is the tumor volume at a certain measurement, and the data of $T_{RTV}$ and $C_{RTV}$ on the same day were used.

TGI (%) reflects the rate of tumor growth inhibition. TGI(%)=[(1-(Average tumor volume at the end of administration for a treatment group-Average tumor volume at the beginning of administration for the treatment group))/(Average tumor volume at the end of treatment for a vehicle control group-Average tumor volume at the beginning of treatment for the vehicle control group)] $\times$ 100.

Tumor weights will be measured at the end of the assay, and $T_{weight}/C_{weight}$ percent will be calculated, wherein $T_{weight}/C_{weight}$ represents tumor weights of the administration group and the vehicle control group, respectively.

3.4 Statistical analysis

Statistical analysis was performed using SPSS software based on the relative tumor volume and tumor weight at the end of the test. T-test was used for comparison between two groups and one-way ANOVA was used for comparison among three or more groups. If variance was not neat (significant difference was found in F value), Games-Howell method was used for test. P<0.05 was considered a significant difference.

4. Assay conclusion

The results were shown in Table 4.

Table 4

| Group | Dose (mpk) | TGI(%) | P value |
|---|---|---|---|
| Compound 4 | 20 | 85.90 | 0.009 |
| | 50 | 97.66 | 0.005 |
| Compound 11 | 20 | 54.16 | 0.091 |
| | 50 | 84.11 | 0.010 |

Conclusion: After 18 days of administration observation, compared with the vehicle control group, the compounds of the present disclosure show remarkable tumor inhibition effects at the administration doses. In addition, the compounds of the present disclosure show a dose-dependent effect, and the tumor inhibition effect in the high dose group is better than that in the low dose group. During the administration period, the animals in all dose groups were well tolerated.

(2) **In vivo efficacy study of the compounds of the present disclosure in a PAN02 tumor model**

**Assay purpose:**

The anti-tumor effect of the test compound was investigated on an in vivo tumor model of mouse pancreatic cancer PAN02.

**Assay method:**

Female C57BL/6 mice were subcutaneously inoculated with PAN02 mouse pancreatic cancer cell line, grouped according to body weight and tumor volume on the 8th day after inoculation, and administered as described below.

Group 1 (control group): Vehicle control was administered intragastrically at a dose of 0.1 mL/10 g body weight twice a day.

Group 2: Compound 4 dissolved in (5% DMSO+40% PEG 400+10% Solutol+45% $DDH_2O$) was administered intragastrically at a dose of 30 mg/kg body weight.

During the assay, the body weight and tumor volume of mice were measured three times a week, and the tumor volume was calculated according to the formula of length $\times$ width$^2$/2. The tumor proliferation rate and tumor inhibition rate were calculated according to the formula: tumor proliferation rate = tumor volume of treatment group/tumor volume of control group $\times$ 100%; tumor inhibition rate = (tumor volume of control group-tumor volume of treatment group)/tumor volume of control group.

Student's t-test was used for statistical analysis between groups, and p<0.05 indicated significant difference.

**Assay results:**

In the mouse PAN02 tumor model, Compound 4 showed anti-tumor activity at a dose of 30 mg/kg with a tumor proliferation rate of 37.61% on day 32. Compared with the control group, Compound 4 showed a significant anti-tumor effect (P=0.0417). The detailed results were shown in Figure 3.

**Assay conclusion:**

The compound of the present disclosure shows excellent anti-tumor effect in a PAN02 model.

(3) **Anti-tumor activity assay of the compound of the present disclosure in an EMT-6 in vivo tumor model**

**Assay purpose:**

The anti-tumor effect of the test compound was investigated on an in vivo tumor model of mouse breast cancer EMT-6.

**Assay method:**

Female BALB/C mice were subcutaneously inoculated with an EMT-6 mouse breast cancer cell line, grouped according to body weight and tumor volume on the 8th day after inoculation, and administered as described below.

Group 1 (control group): Vehicle control was administered intragastrically at a dose of 0.1 mL/10 g body weight twice a day.

Group 2: Compound 4 dissolved in (5% DMSO+40% PEG 400+10% Solutol+45% $DDH_2O$) was administered intragastrically at a dose of 30 mg/kg body weight.

During the assay, the body weight and tumor volume of mice were measured three times a week, and the tumor volume was calculated according to the formula of length $\times$ width$^2$/2. The tumor proliferation rate and tumor inhibition rate were calculated according to the formula: tumor proliferation rate = tumor volume of treatment group/tumor volume of control group $\times$ 100%; tumor inhibition rate = (tumor volume of control group-tumor volume of treatment group)/tumor volume of control group.

Student's t-test was used for statistical analysis between groups, and p<0.05 indicated significant difference.

**Assay results:**

In the mouse EMT-6 tumor model, Compound 4 showed anti-tumor activity at a dose of 30 mg/kg with a tumor proliferation rate of 23.92% on Day 19. At the end of the assay, the tumor proliferation rate reached 36.79%. Compared with the control group, Compound 4 showed a significant anti-tumor effect (P=0.0493). The detailed results were shown in Figure 4.

**Assay conclusion:**

The compound of the present disclosure shows excellent anti-tumor effect in an EMT-6 model.

## Claims

**1.** A compound represented by formula (I), or an isomer or a pharmaceutically acceptable salt thereof,

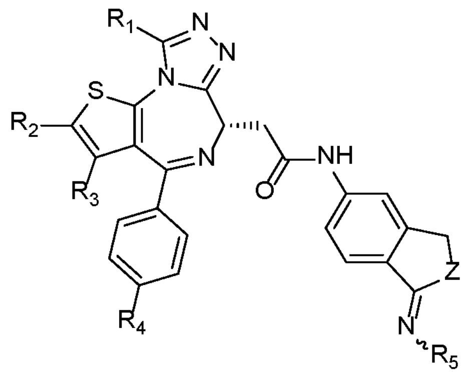

( I )

wherein,

$R_1$, $R_2$ and $R_3$ are each independently selected from $C_{1-3}$ alkyl optionally substituted with 1, 2 or 3 $R_a$;
$R_4$ is selected from the group consisting of H, F, Cl, Br, I, OH, and $NH_2$;
$R_5$ is selected from the group consisting of H, OH, $NH_2$, CN, $C_{1-6}$ alkyl, $C_{1-6}$ alkylamino, $C_{1-6}$ alkoxy and 4- to 6-membered heterocycloalkyl, wherein the $C_{1-6}$ alkyl, $C_{1-6}$ alkylamino, $C_{1-6}$ alkoxy and 4- to 6-membered heterocycloalkyl are each independently optionally substituted with 1, 2 or 3 $R_b$;
Z is selected from the group consisting of O, $NR_6$ and $CHR_6$;
$R_6$ is selected from the group consisting of H and $C_{1-3}$ alkyl optionally substituted with 1, 2 or 3 $R_c$;
or $R_5$, $R_6$ and the atoms to which they are attached together form 5- to 6-membered heterocycloalkenyl or 5- to 6-membered heteroaryl, wherein the 5- to 6-membered heteroalkenyl and 5- to 6-membered heteroaryl are each independently optionally substituted with 1, 2 or 3 $R_d$;
$R_a$, $R_c$, and $R_d$ are each independently selected from the group consisting of F, Cl, Br, I, OH, $NH_2$, and $CH_3$;
$R_b$ is selected from the group consisting of F, Cl, Br, I, OH, $NH_2$, CN, $C_{1-3}$ alkyl and $C_{1-3}$ alkoxy, wherein the $C_{1-3}$ alkyl and $C_{1-3}$ alkoxy are each independently optionally substituted with 1, 2 or 3 R;
R is each independently selected from the group consisting of F, Cl, Br, I, OH, $NH_2$ and $CH_3$;
the 4- to 6-membered heterocycloalkyl, 5- to 6-membered heterocycloalkenyl and 5- to 6-membered heteroaryl each contain 1, 2, 3 or 4 heteroatoms or heteroatom groups independently selected from the group consisting of -NH-, -O-, -S- and N.

**2.** The compound, or an isomer or a pharmaceutically acceptable salt thereof according to claim 1, wherein $R_1$, $R_2$ and $R_3$ are each independently selected from $CH_3$ optionally substituted with 1, 2 or 3 $R_a$.

**3.** The compound, or an isomer or a pharmaceutically acceptable salt thereof according to claim 2, wherein $R_1$, $R_2$ and $R_3$ are each independently selected from $CH_3$.

**4.** The compound, or an isomer or a pharmaceutically acceptable salt thereof according to any one of claims 1-3, wherein $R_4$ is selected from Cl.

**5.** The compound, or an isomer or a pharmaceutically acceptable salt thereof according to any one of claims 1-3, wherein $R_b$ is selected from the group consisting of F, Cl, Br, I, OH, $NH_2$, CN, $CH_3$, $CH_2CH_3$ and $OCH_3$, wherein the $CH_3$, $CH_2CH_3$ and $OCH_3$ are optionally substituted with 1, 2 or 3 R.

**6.** The compound, or an isomer or a pharmaceutically acceptable salt thereof according to claim 5, wherein $R_b$ is selected from the group consisting of F, Cl, Br, I, OH, $NH_2$, CN, $CH_3$, $CH_2F$, $CHF_2$, $CF_3$, $CH_2CH_3$ and $OCH_3$.

**7.** The compound, or an isomer or a pharmaceutically acceptable salt thereof according to any one of claims 1-3, wherein $R_5$ is selected from the group consisting of H, OH, $NH_2$, CN, $C_{1-3}$ alkyl, $C_{1-3}$ alkylamino, $C_{1-3}$ alkoxy and oxetanyl, wherein the $C_{1-3}$ alkyl, $C_{1-3}$ alkylamino, $C_{1-3}$ alkoxy or oxetanyl are each independently optionally substituted with 1, 2 or 3 $R_b$.

**8.** The compound, or an isomer or a pharmaceutically acceptable salt thereof according to claim 7, wherein $R_5$ is

selected from the group consisting of H, OH, $NH_2$, CN, $CH_3$, $CH_2CH_3$, $CH(CH_3)_2$, $OCH_3$, $OCH_2CH_3$, $NH(CH_3)$ and oxetanyl, wherein the $CH_3$, $CH_2CH_3$, $CH(CH_3)_2$, $OCH_3$, $OCH_2CH_3$, $NH(CH_3)$ or oxetanyl are each independently optionally substituted with 1, 2 or 3 $R_b$.

**9.** The compound, or an isomer or a pharmaceutically acceptable salt thereof according to claim 6 or 8, wherein $R_5$ is selected from the group consisting of H, OH, $NH_2$, CN, $CH_3$, $CH_2F$, $CHF_2$, $CF_3$, $CH_2CH_3$, $CH(CH_3)_2$, $OCH_3$, $OCH_2F$, $OCRF_2$, $OCF_3$, $OCH_2CH_3$, $OCH(CH_3)_2$, $OCH_2CH_2OCH_3$, $NH(CH_3)$, $N(CH_3)_2$, $NHCH(CH_3)_2$, and

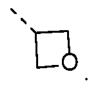

**10.** The compound, or an isomer or a pharmaceutically acceptable salt thereof according to any one of claims 1-3, wherein $R_5$, $R_6$ and the atoms to which they are attached together form 4,5-dihydroisoxazolyl, pyrazolyl, pyrrolyl and imidazolyl, wherein the 4,5-dihydroisoxazolyl, pyrazolyl, pyrrolyl or imidazolyl are each independently optionally substituted with 1, 2 or 3 $R_d$.

**11.** The compound, or an isomer or a pharmaceutically acceptable salt thereof according to claim 10 wherein $R_5$, $R_6$ and the atoms to which they are attached together form

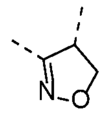

or

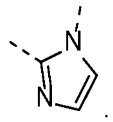

**12.** The compound, or an isomer or a pharmaceutically acceptable salt thereof according to claim 11, wherein the moiety

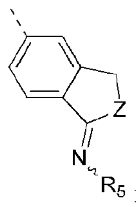

is selected from the group consisting of

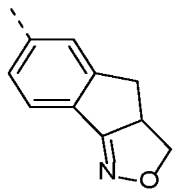

and

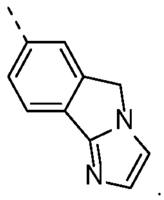

13. The compound, or an isomer or a pharmaceutically acceptable salt thereof according to any one of claims 1-9, wherein the compound is selected from the group consisting of

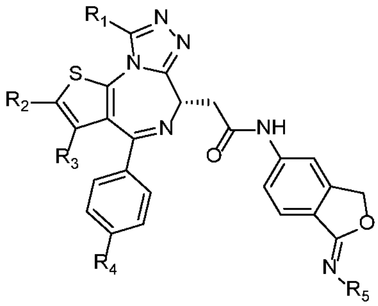

( I -1)

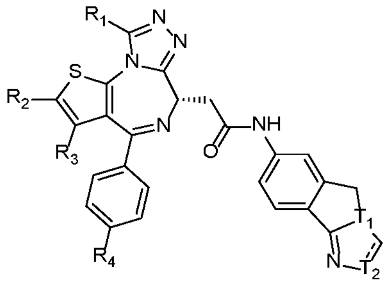

( I -2)

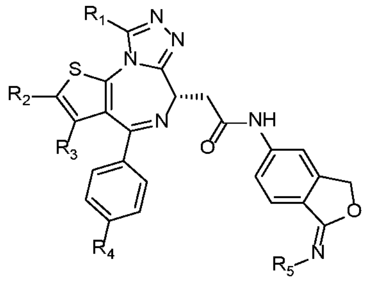

( I -3)

,

wherein,

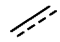

is selected from single bond and double bond;

$R_1$, $R_2$ and $R_3$ are as defined in any one of claims 1, 2 or 3;

$R_4$ is as defined in claim 1 or 4;

$T_1$ is CH or N;

$T_2$ is CH, N, or O;

$R_5$ is as defined in any one of claims 1, 7, 8 or 9.

14. The compound, or an isomer or a pharmaceutically acceptable salt thereof according to claim 13, wherein the compound is selected from the group consisting of

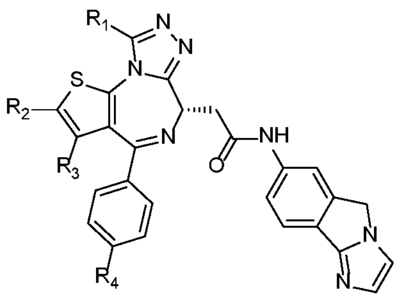

( I -2a)

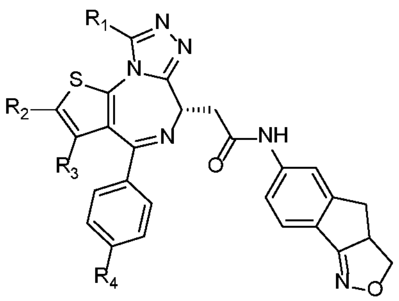

( I -2b)

wherein,

$R_1$, $R_2$, $R_3$ and $R_4$ are as defined in claim 13.

15. A compound represented by the following formula, or an isomer or a pharmaceutically acceptable salt thereof,

wherein the compound is selected from the group consisting of

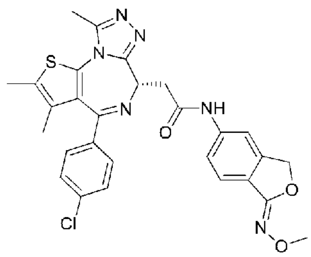
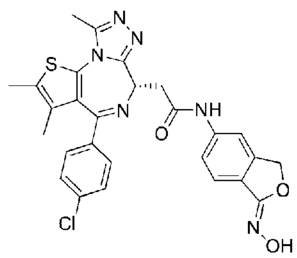
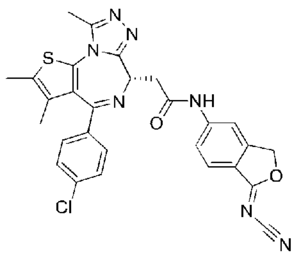
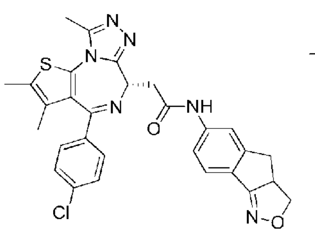
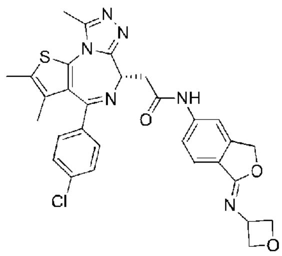
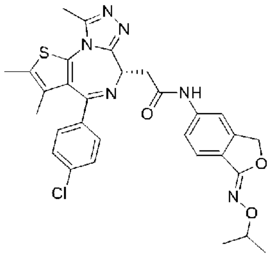
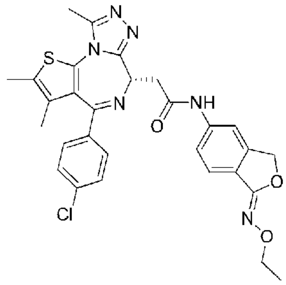
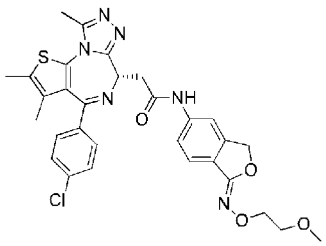
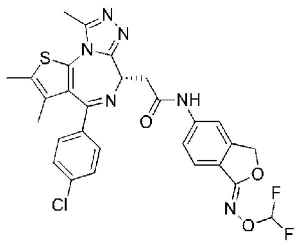
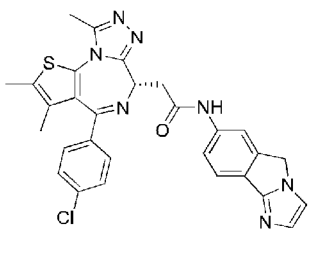
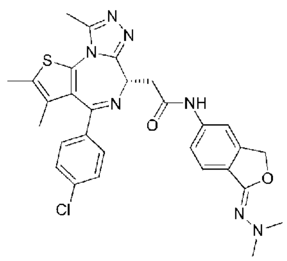
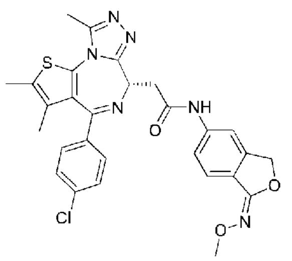
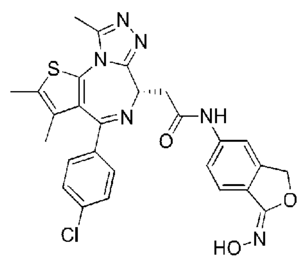
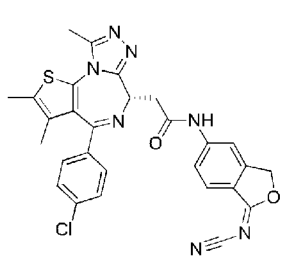
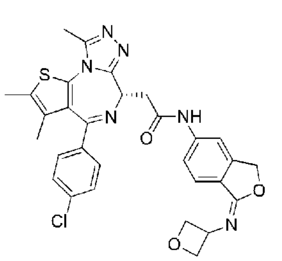

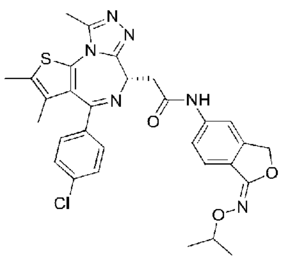
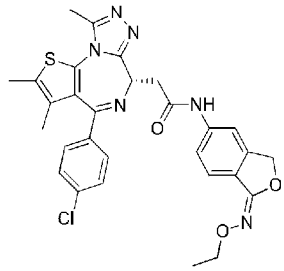
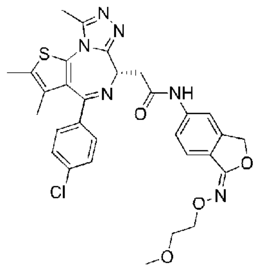

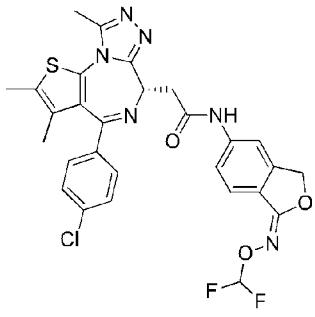
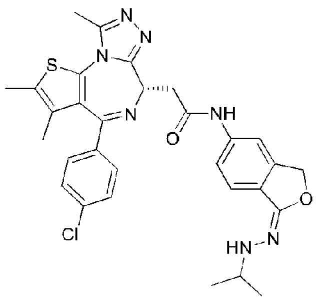
.

16. The compound, or an isomer or a pharmaceutically acceptable salt thereof according to claim 15, wherein the compound is selected from the group consisting of

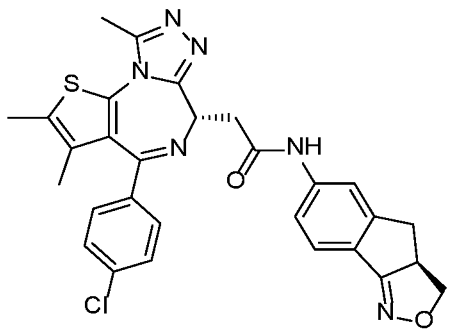
and
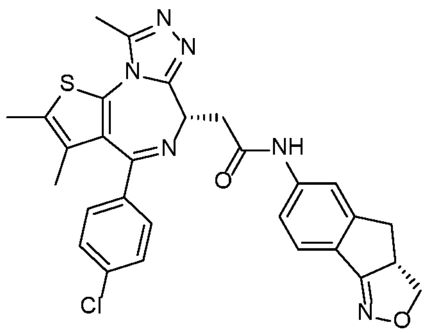
.

17. A pharmaceutical composition comprising a therapeutically effective amount of the compound, or an isomer or a pharmaceutically acceptable salt thereof according to any one of claims 1 to 16 as an active ingredient and pharmaceutically acceptable carrier (s).

18. Use of the compound, or an isomer or a pharmaceutically acceptable salt thereof according to any one of claims 1 to 16, or the composition according to claim 17 in the manufacture of a medicament for the treatment of a disease associated with BET Bromodomain protein inhibitor and PD-L1 gene expression.

19. The use according to claim 18, wherein the medicament is a drug for treating a tumor.

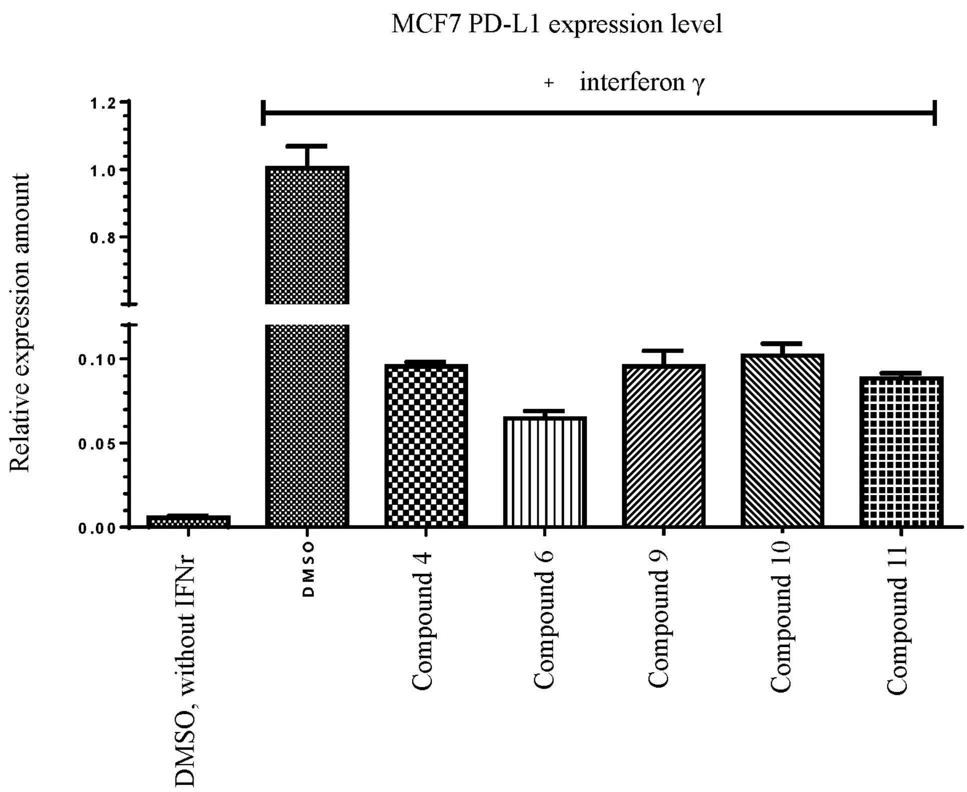
MCF7 PD-L1 expression level
+ interferon γ
Relative expression amount
1.2
1.0
0.8
0.10
0.05
0.00
DMSO, without IFNr
DMSO
Compound 4
Compound 6
Compound 9
Compound 10
Compound 11

FIG. 1

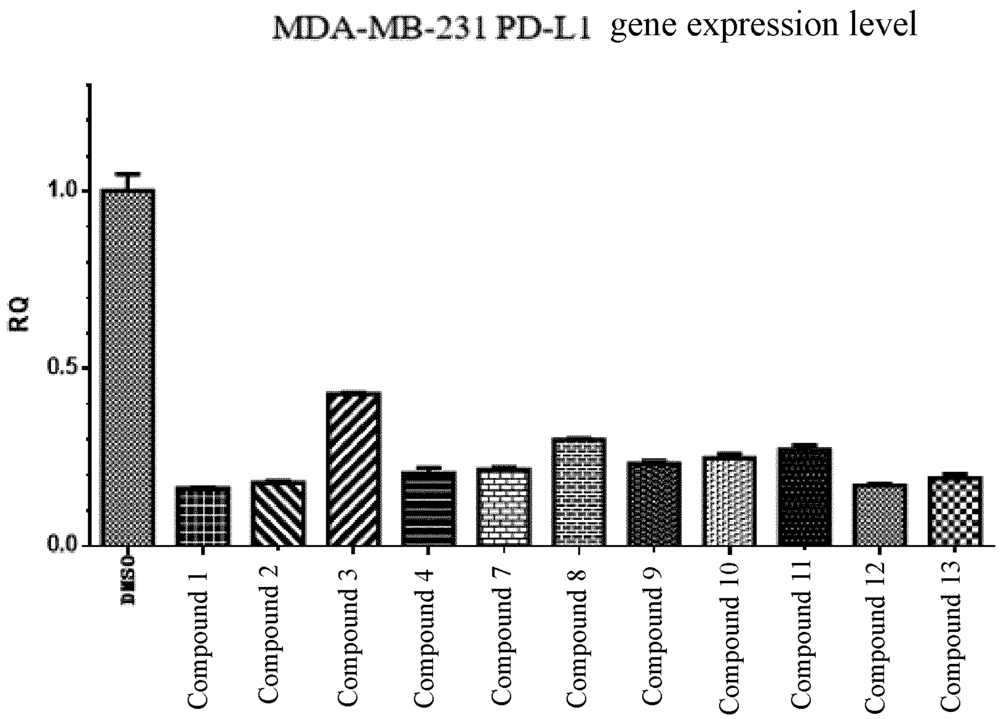
MDA-MB-231 PD-L1 gene expression level
RQ
1.0
0.5
0.0
DMSO
Compound 1
Compound 2
Compound 3
Compound 4
Compound 7
Compound 8
Compound 9
Compound 10
Compound 11
Compound 12
Compound 13

FIG. 2

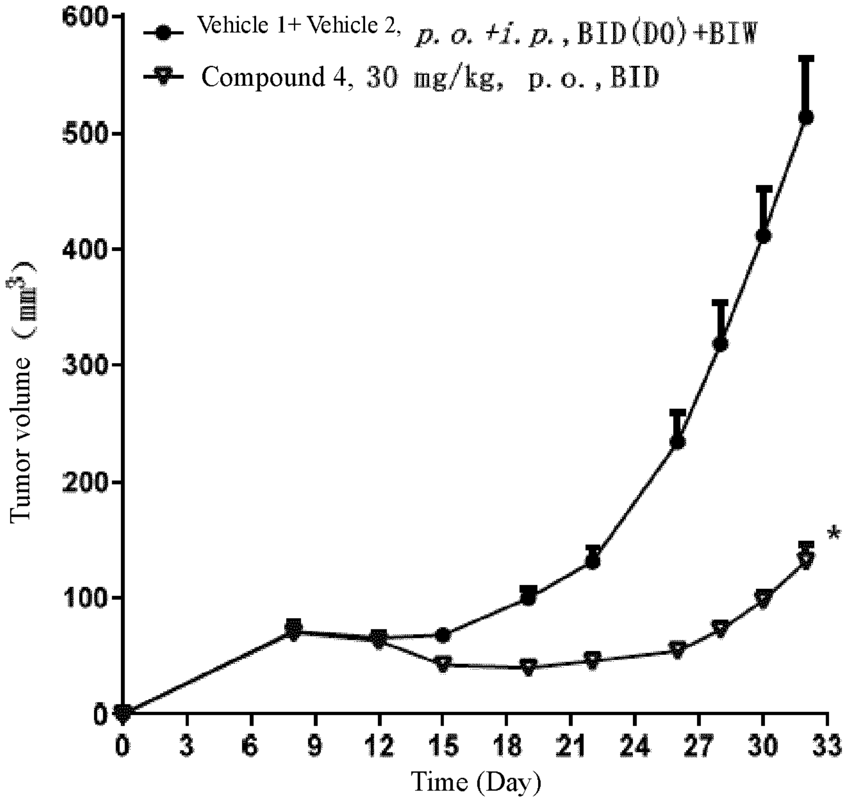
Vehicle 1+ Vehicle 2, p. o. +i. p., BID(D0)+BIW
Compound 4, 30 mg/kg, p. o., BID
Tumor volume (mm³)
Time (Day)
*
600
500
400
300
200
100
0
0
3
6
9
12
15
18
21
24
27
30
33

FIG. 3

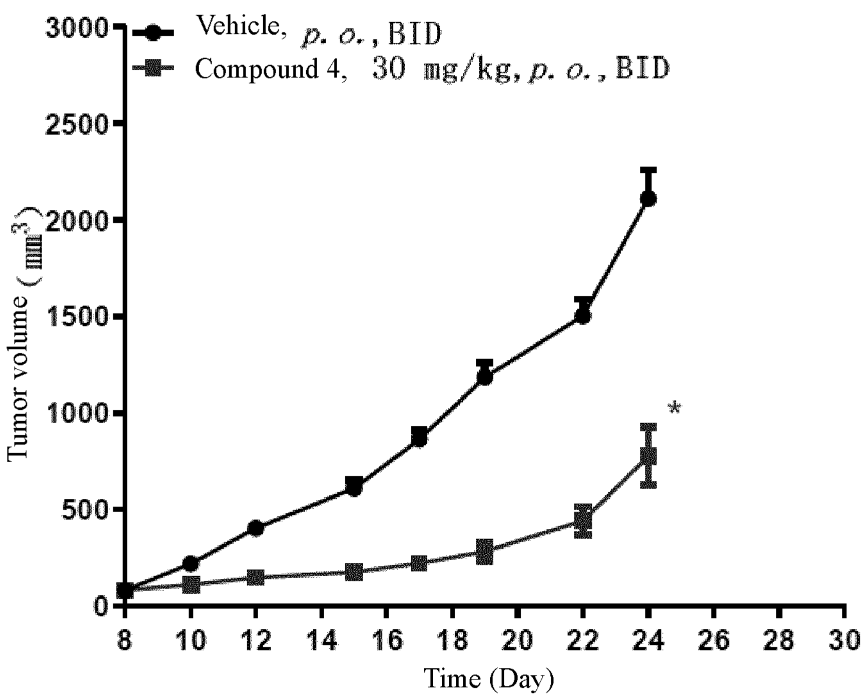
Vehicle, p. o., BID
Compound 4, 30 mg/kg, p. o., BID
Tumor volume (mm³)
Time (Day)
*
3000
2500
2000
1500
1000
500
0
8
10
12
14
16
18
20
22
24
26
28
30

FIG. 4

## INTERNATIONAL SEARCH REPORT

International application No.
**PCT/CN2020/078187**

**A. CLASSIFICATION OF SUBJECT MATTER**

C07D 495/14(2006.01)i; A61K 31/55(2006.01)i; A61P 43/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; CNTXT; SIPOABS; DWPI; STNext: 南京明德新药; 沈春莉; 噻吩并二氮杂卓; 溴结构域; thienotriazolodiazepine; BET; Bromodomain; PD-L1; Birabresib

**C. DOCUMENTS CONSIDERED TO BE RELEVANT**

| Category* | Citation of document, with indication, where appropriate, of the relevant passages | Relevant to claim No. |
|---|---|---|
| PX | WO 2019056950 A1 (MEDSHINE DISCOVERY INC.) 28 March 2019 (2019-03-28)<br>claims 1-19 | 1-19 |
| A | CN 101910182 A (MITSUBISHI TANABE PHARMA CORPORATION) 08 December 2010 (2010-12-08)<br>claims 1-7, abstract, description, page 12, compounds 1-18 | 1-19 |
| A | CN 1237180 A (YOSHITOMI PHARMACEUTICAL CO., LTD.) 01 December 1999 (1999-12-01)<br>claims 1-16 | 1-19 |
| A | US 5712274 A (YOSHITOMI PHARMACEUTICAL IND. KK.) 27 January 1998 (1998-01-27)<br>abstract, and claims 1-12 | 1-19 |
| A | WO 2018144789 A1 (THE REGENTS OF UNIVERSITY OF MICHIGAN) 09 August 2018 (2018-08-09)<br>claims 1-68 | 1-19 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 April 2020** | **28 May 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2020/078187

C. DOCUMENTS CONSIDERED TO BE RELEVANT

| Category* | Citation of document, with indication, where appropriate, of the relevant passages | Relevant to claim No. |
|---|---|---|
| A | LEWIN, J. et al. "Phase Ib Trial With Birabresib, a Small-Molecule Inhibitor of Bromodomain and Extraterminal Proteins, in Patients With Selected Advanced Solid Tumors" *Journal of Clinical Oncology,* Vol. 36, No. 30, 07 May 2018 (2018-05-07), pp. 3007-3018 | 1-19 |

Form PCT/ISA/210 (second sheet) (January 2015)

**INTERNATIONAL SEARCH REPORT**
**Information on patent family members**

International application No.
**PCT/CN2020/078187**

| Patent document cited in search report | | | Publication date (day/month/year) | Patent family member(s) | | | Publication date (day/month/year) |
|---|---|---|---|---|---|---|---|
| WO | 2019056950 | A1 | 28 March 2019 | None | | | |
| CN | 101910182 | A | 08 December 2010 | JP | WO2009084693 | A1 | 19 May 2011 |
| | | | | KR | 20100112596 | A | 19 October 2010 |
| | | | | EP | 2239264 | A1 | 13 October 2010 |
| | | | | US | 2015335656 | A1 | 26 November 2015 |
| | | | | KR | 101600634 | B1 | 07 March 2016 |
| | | | | CA | 2710740 | A1 | 09 July 2009 |
| | | | | CN | 101910182 | B | 17 July 2013 |
| | | | | US | 8476260 | B2 | 02 July 2013 |
| | | | | CA | 2710740 | C | 19 July 2016 |
| | | | | US | 2010286127 | A1 | 11 November 2010 |
| | | | | WO | 2009084693 | A1 | 09 July 2009 |
| | | | | US | 9125915 | B2 | 08 September 2015 |
| | | | | JP | 5478262 | B2 | 23 April 2014 |
| | | | | US | 2013261109 | A1 | 03 October 2013 |
| | | | | EP | 2239264 | A4 | 11 January 2012 |
| CN | 1237180 | A | 01 December 1999 | EA | 001732 | B1 | 27 August 2001 |
| | | | | JP | 3094453 | B2 | 03 October 2000 |
| | | | | HK | 1023571 | A1 | 06 February 2004 |
| | | | | PT | 989131 | E | 31 March 2003 |
| | | | | EP | 0989131 | B1 | 13 November 2002 |
| | | | | DK | 0989131 | T3 | 03 March 2003 |
| | | | | NO | 991191 | D0 | 11 March 1999 |
| | | | | CA | 2265645 | A1 | 19 March 1998 |
| | | | | NO | 991191 | L | 23 April 1999 |
| | | | | NO | 323892 | B1 | 16 July 2007 |
| | | | | KR | 20000036057 | A | 26 June 2000 |
| | | | | NO | 991191 | A | 23 April 1999 |
| | | | | DE | 69717160 | T2 | 08 May 2003 |
| | | | | AT | 227727 | T | 15 November 2002 |
| | | | | ES | 2182108 | T3 | 01 March 2003 |
| | | | | EP | 0989131 | A1 | 29 March 2000 |
| | | | | WO | 9811111 | A1 | 19 March 1998 |
| | | | | EA | 199900287 | A1 | 26 August 1999 |
| | | | | AU | 3786097 | A | 02 April 1998 |
| | | | | AU | 716490 | B2 | 24 February 2000 |
| | | | | DE | 69717160 | D1 | 19 December 2002 |
| | | | | EP | 0989131 | A4 | 29 March 2000 |
| | | | | PT | 989131 | T | 31 March 2003 |
| | | | | KR | 100338144 | B1 | 24 May 2002 |
| | | | | CA | 2265645 | C | 23 January 2007 |
| | | | | CN | 1109037 | C | 21 May 2003 |
| US | 5712274 | A | 27 January 1998 | EP | 0661284 | A4 | 23 May 1995 |
| | | | | KR | 957003566 | A | 20 September 1995 |
| | | | | EP | 0661284 | A1 | 05 July 1995 |
| | | | | WO | 9406802 | A1 | 31 March 1994 |
| WO | 2018144789 | A1 | 09 August 2018 | US | 2019382415 | A1 | 19 December 2019 |
| | | | | EP | 3577120 | A1 | 11 December 2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201910172886